(19) Europäisches Patentamt / European Patent Office / Office européen des brevets

(11) **EP 2 711 026 A1**

(12) **EUROPEAN PATENT APPLICATION**

(43) Date of publication:
**26.03.2014 Bulletin 2014/13**

(51) Int Cl.:
***A61K 51/04*** *(2006.01)*

(21) Application number: **13182326.2**

(22) Date of filing: **30.08.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.09.2012 JP 2012226401**
**27.02.2013 JP 2013052369**

(71) Applicants:
- **Kyoto University**
  **Kyoto-shi, Kyoto 606-8501 (JP)**
- **NIHON MEDI-PHYSICS CO., LTD.**
  **Tokyo 136-0075 (JP)**

(72) Inventors:
- **Saji, Hideo**
  **Kyoto-shi,, Kyoto 606-8501 (JP)**
- **Ono, Masahiro**
  **Kyoto-shi,, Kyoto 606-8501 (JP)**
- **Ihara, Masafumi**
  **Kyoto-shi,, Kyoto 606-8501 (JP)**
- **Seki, Ikuya**
  **Kyoto-shi,, Tokyo 136-0075 (JP)**

(74) Representative: **TBK**
**Bavariaring 4-6**
**80336 München (DE)**

(54) **Radioactive fluorine-labeled quinoxaline compound**

(57) Provided is a compound effective as a diagnostic imaging probe targeting amyloid and an agent for Alzheimer's disease diagnosis including the compound.

FIG. 1

SCHEME 1

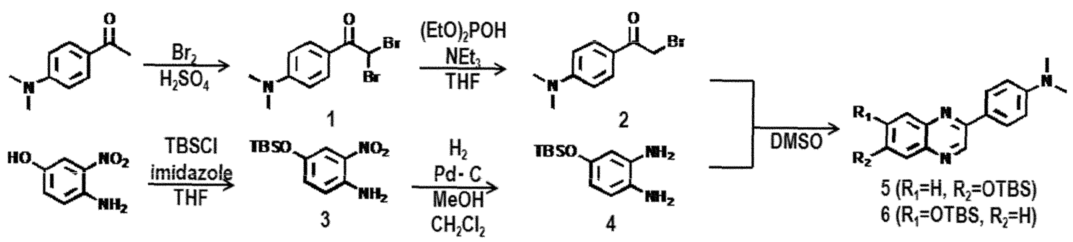


SCHEME 2

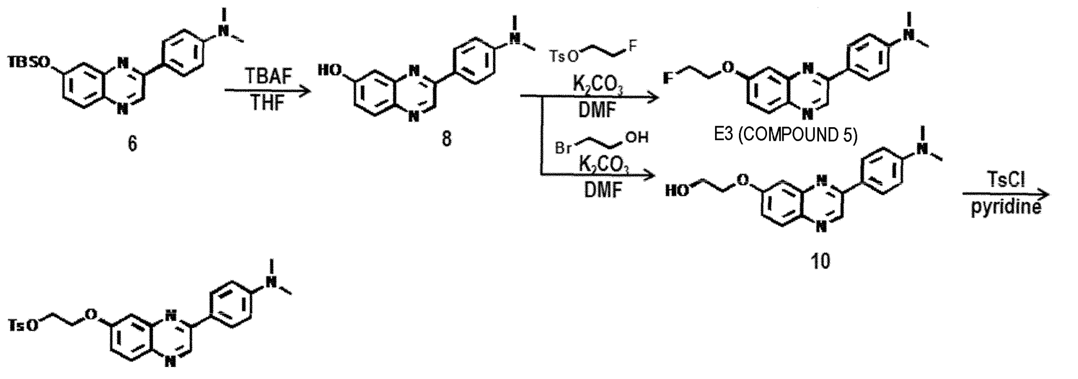


COMPOUND 3

EP 2 711 026 A1

Printed by Jouve, 75001 PARIS (FR)

## Description

CROSS-REFERECE TO RELATED APPLICATIONS

The disclosure of Japanese Patent Application No. 2012-226401 filed on September 25, 2012 and Japanese Patent Application No.2013-52369 filed on February 27, 2013 including the specifications, drawings and abstracts are incorporated hereinto by reference.

BACKGROUND

The present invention relates to a radioactive fluorine-labeled quinoxaline compound. More specifically, it relates to a compound which is used for the diagnosis of a degenerative disease of the head and is useful for the detection of amyloid in focal sites in the diagnosis of a disease in which amyloid accumulates, including Alzheimer's disease.

Diseases occurring by the deposition of a fibrous protein called amyloid in various organs or tissues in the body are collectively called amyloidosis. Common to amyloidosis is that a fibrous protein rich in a β-sheet structure, called amyloid deposits in organs or foci throughout the body and causes dysfunction in the organs or tissues.

Alzheimer's disease (hereinafter referred to as AD), a representative disease of amyloidosis, is known as a disease causing dementia. This disease can be said to be a disease of high social concern compared to other amyloidosis because it is a disease leading to death by the gradually progressive deposition of amyloid in the brain. In developed countries, the number of AD patients has rapidly increased with the aging of society in recent years, which becomes a social problem.

According to a histopathological standpoint, AD is characterized by 3 pathological findings in the brain: the occurrence of senile plaques, neurofibrillary tangles, and expanded neuronal loss. A senile plaque is a structure containing amyloid as the major component, and is considered to be a pathological finding in the brain occurring at the earliest stage in the development of AD, specifically 10 years or more before the occurrence of clinical symptoms.

The diagnosis of AD is made by carrying out various cognitive function evaluations (for example, Hasegawa's scale, ADAS-JCog, and MMSE) supplementarily combined with diagnostic imaging, such as CT and MRI. However, these methods based on such cognitive function evaluations have disadvantages that they are low in the sensitivity of diagnosis at the early stage of the occurrence of the disease and further that the results of diagnosis are affected by cognitive functions which each individual genuinely has.

The amyloid constituting a senile plaque is reported to be an aggregate of amyloid β-proteins (hereinafter referred to as Aβ), and many studies further report that the aggregate of Aβ causes nerve cell toxicity when being in a β-sheet structure. Based on these findings, the so-called "amyloid cascade hypothesis" has been proposed that the deposition of Aβ in the brain triggers the formation of neurofibrillary tangles and neuronal loss as events downstream thereof (G. McKhann et al., "Clinical diagnosis of Alzheimer's disease: Report of the NINCDS-ADRDA Work Group under the auspices of Department of Health and Human Services Task Force on Alzheimer's Disease.", Neurology, 1984, 34, p.939-944).

Based on these facts, attempts have been made to detect AD in vivo by using a compound having high affinity to amyloid as a marker in recent years (Mengchao Cui et al., "Novel quinoxaline derivatives for in vivo imaging of β-amyloid plaques in the brain", Bioorganic & Medicinal Chemistry Letters, 2011, 21, p.4193-4196).

SUMMARY

As described above, various compounds are disclosed as diagnostic imaging probes for amyloid, and studies are underway towards their clinical application. However, there not yet exists a compound confirmed to have performance acceptable for clinical use.

Made in view of the above-described circumstances, the present invention has an object of obtaining a compound effective as a diagnostic imaging probe targeting amyloid and an agent for amyloidosis diagnosis comprising the compound.

In one embodiment, there is provided a compound represented by the following general formula (1) or a salt thereof:

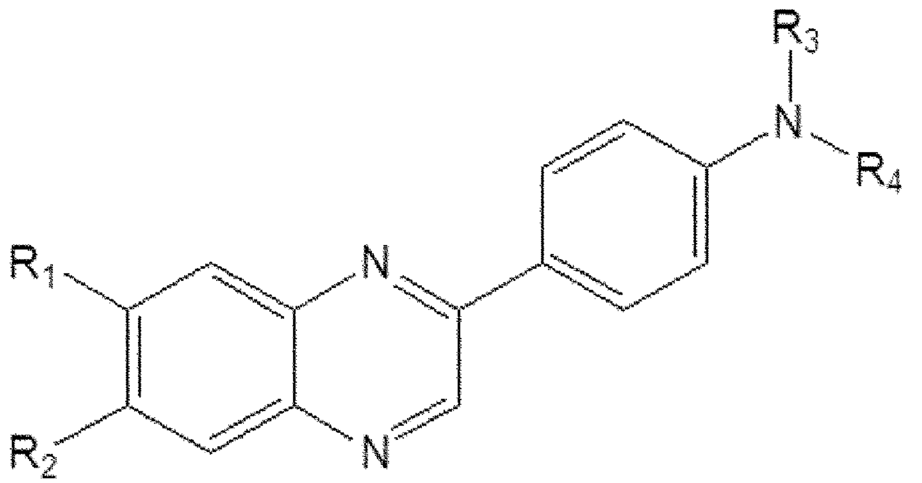

(1)

wherein one of $R_1$ and $R_2$ represents a 2-[$^{18}$F]fluoroethoxy group and the other represents a hydrogen atom; $R_3$ and $R_4$ each independently represent a hydrogen atom or a methyl group with the proviso that the cases where $R_3$ and $R_4$ each represent a hydrogen atom; and where $R_1$ represents a 2-[$^{18}$F]fluoroethoxy group, $R_2$ and $R_3$ each represent a hydrogen atom, and $R_4$ represents a methyl group are excluded,

and an agent for amyloidosis diagnosis, comprising a compound represented by the general formula (1) as an active ingredient.

In another embodiment, there is provided a compound represented by the following general formula (6):

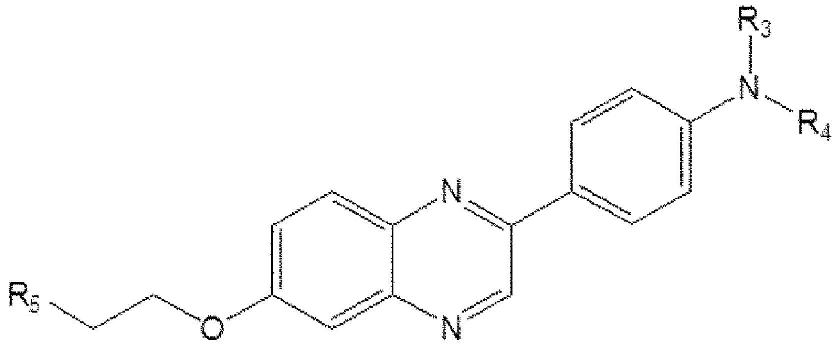

(6)

wherein $R_3$ and $R_4$ each independently represent a hydrogen atom or a methyl group (with the proviso that the case where $R_3$ and $R_4$ each represent a hydrogen atom is excluded), and $R_5$ represents a methanesulfonyloxy group, a trifluoromethanesulfonyloxy group, or an aromatic sulfonyloxy group,

or (7):

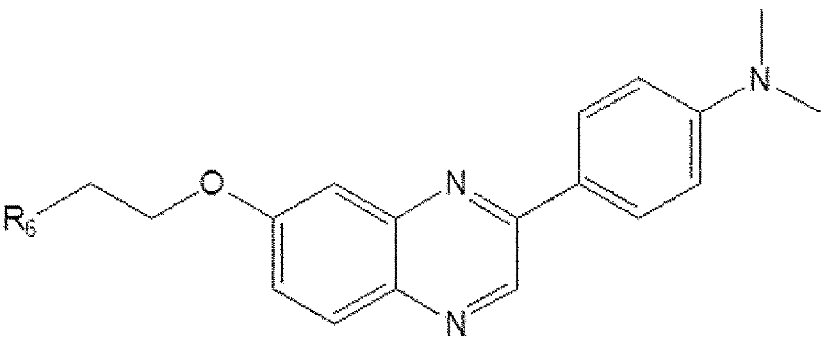

(7)

wherein $R_6$ represents a methanesulfonyloxy group, a trifluoromethanesulfonyloxy group, or an aromatic sulfonyloxy group,

or a salt thereof.

According to the above-mentioned embodiments of the invention, it is possible to provide a new compound having affinity for amyloid and an agent for amyloidosis diagnosis which have good amyloid-visualizing capabilities in vivo.

BRIEF DESCRIPTION OF THE DRAWINGS

The above and other objects, advantages and features of the present invention will be more apparent from the following description of certain preferred embodiments taken in conjunction with the accompanying drawings, in which:

FIG. 1 is a diagram showing synthetic schemes for a compound according to the present embodiment and related compounds thereof;
FIG. 2 is a diagram showing synthetic schemes for a compound according to the present embodiment and related compounds thereof;
FIG. 3 is a diagram showing synthetic schemes for a compound according to Comparative Example and related compounds thereof;
FIG. 4 is a diagram showing synthetic schemes for a compound according to Comparative Example and related compounds thereof;
FIG. 5 is a diagram showing synthetic schemes for a compound according to Comparative Example and related compounds thereof;
FIG. 6 is a picture showing autoradiography of a brain section of an Alzheimer's disease patient using a compound according to the present embodiment ([$^{18}$F]E3, compound 1);
FIG. 7 is a picture showing autoradiography of a brain section of an Alzheimer's disease patient using a compound according to the present embodiment ([$^{18}$F]D3, compound 2);
FIG. 8 is a picture showing autoradiography of a brain section of an Alzheimer's disease patient using a compound according to Comparative Example ([$^{18}$F]E1, compound 7);
FIG. 9 is a picture showing autoradiography of a brain section of an Alzheimer's disease patient using a compound according to Comparative Example ([$^{18}$F]E2, compound 8);
FIG. 10 is a picture showing an immunostaining image of a brain section of an Alzheimer's disease patient;
FIG. 11A and FIG. 11B are pictures showing evaluation results using the brain of a Tg2576 mouse (24-month old, female). FIG. 11A is a picture showing ex vivo autoradiography of [$^{18}$F]D3 (compound 2), and FIG. 11B is a picture showing a thioflavin-stained image;
FIG. 12A and 12B are pictures showing evaluation results using the brain of a normal mouse (24-month old, female). FIG. 12A is a picture showing ex vivo autoradiography of [$^{18}$F]D3 (compound 2), and FIG. 12B is a picture showing a thioflavin-stained image;
FIG. 13 is a diagram showing synthetic schemes for compounds according to the present embodiment and Comparative Example and related compounds thereof;
FIG. 14 is a diagram showing synthetic schemes for related compounds of a compound according to Comparative Example;
FIG. 15 is a diagram showing synthetic schemes for related compounds of compounds according to Example;
FIG. 16 is a picture showing autoradiography of a brain section of an Alzheimer's disease patient using a compound according to Comparative Example ([$^{18}$F]D1);
FIG. 17 is a picture showing autoradiography of a brain section of an Alzheimer's disease patient using a compound according to the present embodiment ([$^{18}$F]D2); and
FIG. 18A and 18B are pictures showing brain sections of an Alzheimer's disease patient. FIG. 18A is a magnified picture showing autoradiography of a compound according to the present invention ([$^{18}$F]D2), and FIG. 18B is a picture showing an immunostaining image.

DETAILED DESCRIPTION

The invention will be now described herein with reference to illustrative embodiments. Those skilled in the art will recognize that many alternative embodiments can be accomplished using the teachings of the present invention and that the invention is not limited to the embodiments illustrated for explanatory purposes.
According to one aspect of the present embodiment, provided are a compound represented by the following formula (3):

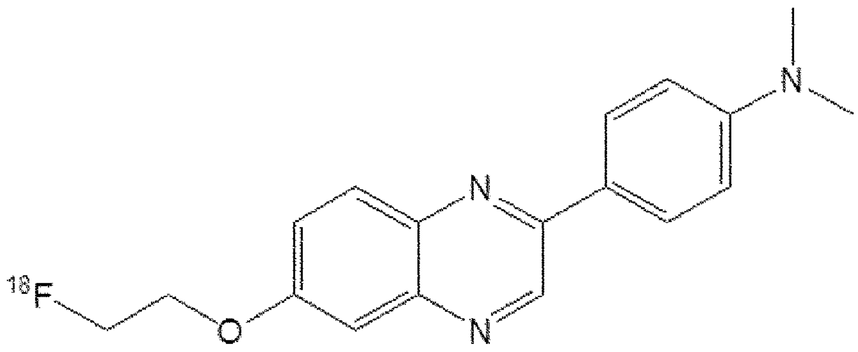

(3)

or formula (4):

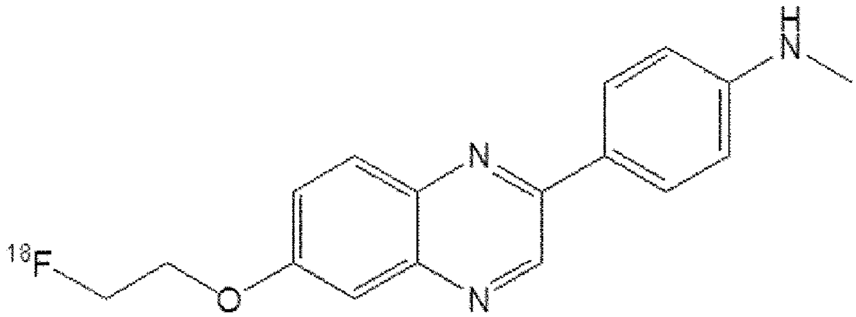

(4)

or formula (5):

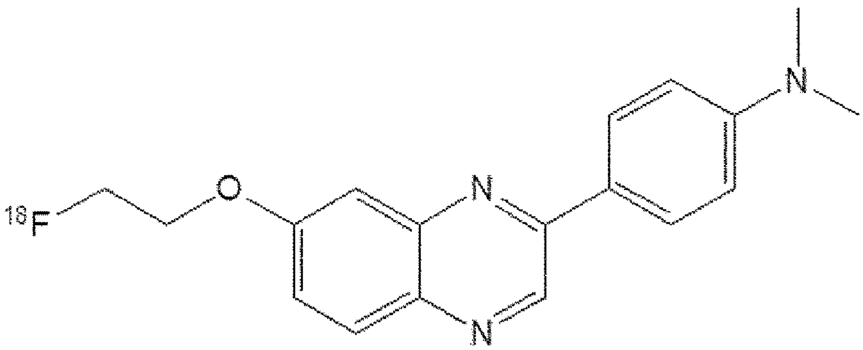

(5)

or a salt thereof, and an agent for amyloidosis diagnosis, comprising the compound (3), (4), or (5) as an active ingredient.

These compounds (3), (4), and (5) are novel and have affinity for amyloid; thus, they can be used as active ingredients for agents for detecting amyloid in vivo.

According to another aspect of the present embodiment, provided is a compound represented by the following formula (7):

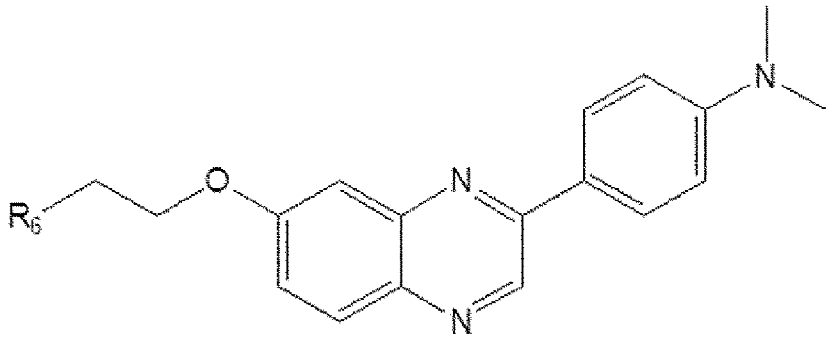

(7)

or formula (8):

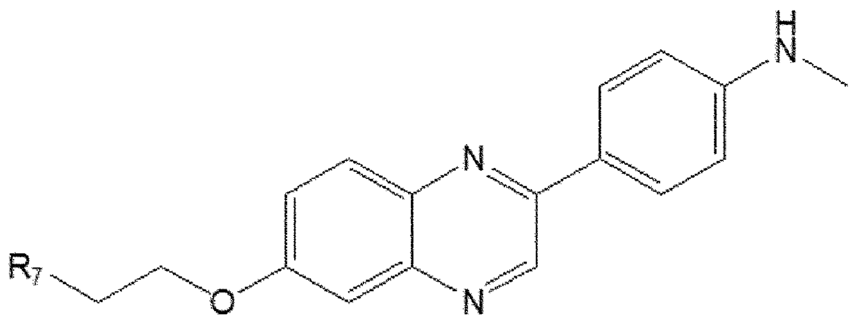

(8)

or formula (9)

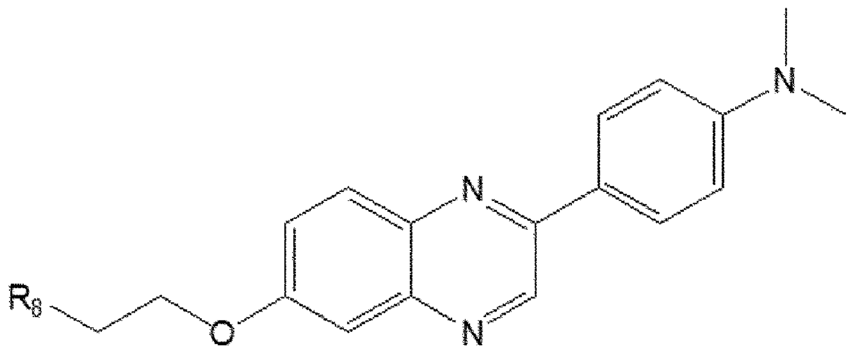

(9)

or a salt thereof. $R_6$, $R_7$, and $R_8$ may each be selected from groups capable of being used as substituents in introducing radioactive fluorine by nucleophilic substitution reaction, may each be preferably selected from sulfonyloxy groups such as a methanesulfonyloxy group, a trifluoromethanesulfonyloxy group, and an aromatic sulfonyloxy, and may each be more preferably an aromatic sulfonyloxy group such as a benzenesulfonyloxy group, a p-nitrobenzenesulfonyloxy group, or a p-toluenesulfonyloxy group, further more preferably a p-toluenesulfonyloxy group.

These functional groups are known to behave as highly eliminable substituents, and are functional groups generally used in the introduction of fluorine using nucleophilic substitution reaction. Thus, the compound (7) can be used as a labeling precursor for a radioactive fluorine-labeled compound according to the present embodiment (compound (3)); the compound (8) can be used as a labeling precursor for a radioactive fluorine-labeled compound according to the present embodiment (compound (4)); and the compound (9) can be used as a labeling precursor for a radioactive fluorine-labeled compound according to the present embodiment (compound (5)).

The compounds (7), (8), and (9) as precursors for radioactive fluorine-labeled compounds according to the present embodiment can each be synthesized by synthesizing a compound in which a hydroxyl group is introduced at the position at which a sulfonic acid ester group is to be introduced and reacting the resultant with a halogenated sulfonyl according to the purpose or the sulfonic acid anhydride concerned.
The compound in which a hydroxyl group is introduced at the position at which a sulfonic acid ester group is to be introduced can be readily synthesized by those of ordinary skill in the art, based on a known method (for example,

methods described in the documents "Mengchao Cui et al., Bioorganic & Medicinal Chemistry Letters, 2011, 21, p.4193-4196" and "Lisheng Mao et al., Synthesis, 2004, 15, 2535-2539").
Radioactive fluorine-labeled compounds according to the present embodiment (compounds (3), (4), and (5)) can be synthesized by a method known to those of ordinary skill in the art, such as reacting each of the compounds (3), (4), and (5) with [$^{18}$F]fluoride ion in the presence of a phase-transfer catalyst and potassium carbonate.

Like other generally known radioactive diagnostic agents, the agent for amyloidosis diagnosis according to the present embodiment can be prepared as a liquid in which a radioactive fluorine-labeled compound according to the present embodiment is mixed in water or saline, if necessary, adjusted to an appropriate pH, Ringer's solution, or the like. The concentration of the compound should be not more than the concentration providing the stability of the mixed compound.
Its dose as an agent for amyloidosis diagnosis need not be particularly limited provided that it is a concentration sufficient for imaging the distribution of the administered agent. Specifically, about 50 to 600 MBq per adult weighing 60 kg can be used by intravenous administration or local administration. The distribution of the administered agent can be imaged by a known method using a PET apparatus.

## EXAMPLES

The present embodiment will be described below in further detail by giving Examples and Comparative Examples.
For purposes of description, the names of the compounds used in Examples and Comparative Examples are defined as stated in Table 1.

Table 1 Name of Evaluation Compound Used in Examples

| Compound Name | Common Name |
|---|---|
| [$^{18}$F]E3 (Compound 1) | 4-(7-(2-[$^{18}$F]Fluoroethoxy)quinoxalin-2-yl)-N,N-dimethylaniline |
| [$^{18}$F]D3 (Compound 2) | 4-(6-(2-[$^{18}$F]Fluoroethoxy)quinoxalin-2-yl)-N,N-dimethylaniline |
| Compound 3 | 2-((3-(4-(Dimethylamino)phenyl)quinoxalin-6-yl)oxy)ethyl-4-methylbenzenesulfonate |
| Compound 4 | 2-((2-(4-(Dimethylamino)phenyl)quinoxalin-6-yl)oxy)ethyl-4-methylbenzenesulfonate |
| E3 (Compound 5) | 4-(7-(2-Fluoroethoxy)quinoxalin-2-yl)-N,N-dimethylaniline (Non-Radioactive Fluorine-Labeled Form of Compound 1) |
| D3 (Compound 6) | 4-(6-(2-Fluoroethoxy)quinoxalin-2-yl)-N,N-dimethylaniline (Non-Radioactive Fluorine-Labeled Form of Compound 2) |
| [$^{18}$F]E1 (Compound 7) | 4-(7-(2-[$^{18}$F]Fluoroethoxy)quinoxalin-2-yl)aniline |
| [$^{18}$F]E2 (Compound 8) | 4-(7-(2-[$^{18}$F]Fluoroethoxy)quinoxalin-2-yl)-N-methylaniline |
| Compound 9 | 2-((3-(4-((Di-tert-butoxycarbonyl)amino)phenyl)quinoxalin-6-yl)oxy)ethyl-4-methylbenzenesulfonate |
| Compound 10 | 2-((3-(4-((tert-Butoxycarbonyl)(methyl)amino)phenyl)quinoxalin-6-yl)oxy)-ethyl-4-methylbenzenesulfonate |
| E1 (Compound 11) | 4-(7-(2-Fluoroethoxy)quinoxalin-2-yl)aniline (Non-Radioactive Fluorine-Labeled Form of Compound 7) |
| E2 (Compound 12) | 4-(7-(2-Fluoroethoxy)quinoxalin-2-yl)-N-methylaniline (Non-Radioactive Fluorine-Labeled Form of Compound 8) |

In synthesis examples for compounds described in the following Examples and Comparative Examples, each step in the compound synthesis was performed a plurality of times as needed to secure necessary amounts in using as intermediates or the like in other syntheses.
Examples described below are preferred examples and not intended to limit the scope of the present invention.

Example 1: Synthesis of 2-((3-(4-(Dimethylamino)phenyl)quinoxalin-6-yl)oxy)ethyl-4-methylbenzenesulfonate (Compound 3)

The compound 3 as a labeling precursor for [$^{18}$F]E3 was synthesized as follows according to the schemes 1 and 2 shown in FIG. 1.

Step 1-1: Synthesis of 2,2-Dibromo-1-(4-dimethylamino)phenyl)ethanone (1)

1-(4-(Dimethylamino)phenyl)ethanone (3.5 g, 21.5 mmol) was dissolved in concentrated sulfuric acid, which was then stirred at 0°C for 0.5 hour. Bromine (1.12 mL, 21.8 mmol) was slowly added thereto at 0°C, which was then stirred at room temperature overnight. The reaction solution was added to water cooled with ice, and the resultant solid was collected by filtration and washed with water cooled with ice to provide (1) in the scheme 1 (yield: 4.89 g (71.5%)).
$^1$H-NMR (400 MHz, deuterated chloroform) δ 7.98 (d, J=8.8 Hz, 2H), 6.69-6.66 (m, 3H), 3.10 (s, 6H).

Step 1-2: Synthesis of 2-Bromo-1-(4-(dimethylamino)phenyl)ethanone (2)

To tetrahydrofuran (40 mL) was added 2,2-dibromo-1-(4-(dimethylamino)phenyl)ethanone (6.84 g, 21.5 mmol) synthesized in the step 1-1, to which was then slowly added a solution of diethyl phosphite (3 mL) and triethylamine (3.2 mL) in tetrahydrofuran (25 mL) at 0°C through a dropping funnel. The resultant was stirred at room temperature for 11 hours. The solvent was distilled off under reduced pressure for concentration, and the residue was added to water cooled with ice, which was then stirred for 0.5 hour. The resultant solid was collected by filtration and washed with water cooled with ice to provide (2) in the scheme 1 (yield: 3.38 g (65.5%)).
$^1$H-NMR (400 MHz, deuterated chloroform) δ 7.89 (d, J=8.8 Hz, 2H), 6.66 (d, J=8.8 Hz, 2H), 4.36 (s, 2H), 3.08 (s, 6H).

Step 1-3: Synthesis of 4-((tert-Butyldimethylsilyl)oxy)-2-nitroaniline (3)

4-Amino-3-nitrophenol (2.82 g, 18.3 mmol) was dissolved in tetrahydrofuran (50 mL), to which were then added tert-butyldimethylsilyl chloride (3.60 g, 24.0 mmol) and imidazole (2.00 g, 24.0 mmol), followed by stirring the resultant at room temperature for 1 hour. The reaction was stopped by adding water (100 mL); after separation and extraction with diethyl ether (100 mL × 3), the organic layer was dehydrated and dried with magnesium sulfate; and the solvent was distilled off under reduced pressure. The resultant residue was purified by silica-gel chromatography (ethyl acetate/hexane = 1/3 (volume ratio)) to provide (3) in the scheme 1 (yield: 4.81 g (98.1%)).
$^1$H-NMR (400 MHz, deuterated chloroform) δ 7.56 (d, J=2.8 Hz, 1H), 6.97 (dd, J=9.2, 2.8 Hz, 1H), 6.70 (d, J=8.8 Hz, 1H), 5.79 (s, 2H), 0.98 (s, 9H), 0.19 (s, 6H).

Step 1-4: Synthesis of 4-((tert-Butyldimethylsilyl)oxy)benzene-1,2-diamine (4)

4-((tert-Butyldimethylsilyl)oxy)-2-nitroaniline (9.57 g, 35.7 mmol) synthesized in the step 1-3 was dissolved in a mixed solvent of methanol (80 mL) and dichloromethane (40 mL), to which palladium carbon (3.00 g) was then added. The resultant was stirred at room temperature for 7 hours under hydrogen filling. The resultant was filtered with celite, and the filtrate was distilled off under reduced pressure. The residue was purified by silica-gel chromatography (ethyl acetate/hexane = 2/1 (volume ratio)) to provide (4) in the scheme 1 (yield: 7.28 g (85.6%)).
$^1$H-NMR (400 MHz, deuterated chloroform) δ 6.55 (d, J=8.0 Hz, 1H), 6.24 (d, J=2.8 Hz, 1H), 6.18 (dd, J=8.0, 2.4 Hz, 1H), 3.19 (s, 4H), 0.96 (s, 9H), 0.15 (s, 6H).

Step 1-5: Synthesis of 4-(7-((tert-Butyldimethylsilyl)oxy)quinoxalin-2-yl)-N,N-dimethylaniline (6)

In dimethyl sulfoxide (10 mL) was dissolved 4-((tert-butyldimethylsilyl)oxy)benzene-1,2-diamine (476.3 mg, 2 mmol) synthesized in the step 1-4, to which was then added 2-bromo-1-(4-(dimethylamino)phenyl)ethanone (482.0 mg, 2 mmol) synthesized in the step 1-2. The resultant was stirred at room temperature for 2 hours, and after separation and extraction with ethyl acetate (100 mL × 2), the organic layer was dehydrated and dried with magnesium sulfate. The solvent was distilled off under reduced pressure, and the resultant residue was purified by silica-gel chromatography (ethyl acetate/hexane = 1/3 (volume ratio)) to provide (6) in the scheme 1. Here, a fraction with Rf = 0.38 was separated (yield: 161.8 mg (21.3%)).
$^1$H-NMR (400 MHz, deuterated chloroform) δ 9.12 (s, 1H), 8.10 (d, J=8.4 Hz, 2H), 7.91 (d, J=9.2 Hz, 1H), 7.42 (s, 1H), 7.22 (dd, J=2.4, 0.8 Hz, 1H), 6.84 (d, J=8.8 Hz, 2H), 3.07 (s, 6H), 1.04 (s, 9H), 0.30 (s, 6H).

Step 1-6: Synthesis of 3-(4-(Dimethylamino)phenyl)quinoxalin-6-ol (8)

In tetrahydrofuran (10 mL) was dissolved 4-(7-((tert-butyldimethylsilyl)oxy)quinoxalin-2-yl)-N,N-dimethylaniline (161.8 mg, 0.427 mmol) synthesized in the step 1-5, to which tetra-n-butylammonium fluoride (1 mol/L, tetrahydrofuran solution) (512 μL, 0.512 mmol) was then slowly added at 0°C. The resultant was stirred at room temperature for 0.5 hour, and after separation and extraction with ethyl acetate (60 mL × 2), the organic layer was dehydrated and dried with magnesium sulfate. The solvent was distilled off under reduced pressure, and the resultant residue was purified by silica-gel chromatography (ethyl acetate/hexane = 1/1 (volume ratio)) to provide (8) in the scheme 2 (yield: 56.0 mg (49.5%)).
$^1$H-NMR (400 MHz, DMSO-d6) δ 9.22 (s, 1H), 8.17 (d, J=8.8 Hz, 2H), 7.85 (d, J=9.2 Hz, 1H), 7.26 (d, J=8.4 Hz, 1H), 7.21 (s, 1H), 6.86 (d, J=8.8 Hz, 2H), 3.02 (s, 6H).

Step 1-7: Synthesis of 2-((3-(4-(Dimethylamino)phenyl)quinoxalin-6-yl)oxy)ethanol (10)

In N,N-dimethylformamide (10 mL) was dissolved 3-(4-(dimethylamino)phenyl)quinoxalin-6-ol (95.6 mg, 0.361 mmol) synthesized in the step 1-6, to which potassium carbonate (99.8 mg, 0.722 mmol) was then added. The resultant was heat-stirred at 105°C for 0.5 hour, and 2-bromoethanol (51.2 μL, 0.722 mmol) was added thereto, which was then heat-stirred at 105°C overnight. The reaction solution was cooled down to room temperature, and after separation and extraction with ethyl acetate (60 mL × 2), the organic layer was dehydrated and dried with magnesium sulfate. The solvent was distilled off under reduced pressure, and the resultant residue was purified by silica-gel chromatography (ethyl acetate/hexane = 4/1 (volume ratio)) to provide (10) in the scheme 2 (yield: 59.3 mg (53.1%)).
$^1$H-NMR (400 MHz, deuterated chloroform) δ 9.13 (s, 1H), 8.11 (d, J=9.2 Hz, 2H), 7.93 (d, J=9.2 Hz, 1H), 7.39 (s, 1H), 7.32 (d, J=8.8 Hz, 1H), 6.84 (d, J=8.8 Hz, 2H), 4.27 (d, J=4.8 Hz, 2H), 4.07 (d, J=4.0 Hz, 2H), 3.07 (s, 6H).

Step 1-8: Synthesis of 2-((3-(4-(Dimethylamino)phenyl)quinoxalin-6-yl)oxy)ethyl-4-methylbenzenesulfonate (Compound 3)

In pyridine (10 mL) was dissolved 2-((3-(4-(dimethylamino)phenyl)quinoxalin-6-yl)oxy)ethanol (58.4 mg, 0.189 mmol) synthesized in the step 1-7, to which para-toluenesulfonyl chloride (108.1 mg, 0.567 mmol) was then added at 0°C. The resultant was stirred at room temperature overnight, and para-toluenesulfonyl chloride (108.1 mg, 0.567 mmol) was further added thereto, which was then stirred at room temperature for 12 hours. The solvent was distilled off under reduced pressure, and separation and extraction were performed with ethyl acetate (60 mL × 2). After dehydrating and drying the organic layer with magnesium sulfate, the solvent was distilled off under reduced pressure. The resultant residue was purified by silica-gel chromatography (ethyl acetate/hexane = 2/1 (volume ratio)) to synthesize the desired 2-((3-(4-(dimethylamino)phenyl)quinoxalin-6-yl)oxy)ethyl-4-methylbenzenesulfonate (hereinafter, also referred to as "compound 3") (yield: 22.2 mg (25.4%)). $^1$H-NMR (400 MHz, deuterated chloroform) δ 9.13 (s, 1H), 8.10 (d, J=8.8 Hz, 2H), 7.89 (d, J=9.2 Hz, 1H), 7.84 (d, J=8.4 Hz, 1H), 7.33 (d, J=8.8 Hz, 2H), 7.24 (s, 1H), 7.16 (dd, J=9.2, 2.8 Hz, 1H), 6.84 (d, J=8.8 Hz, 2H), 4.48 (d, J=4.8 Hz, 2H), 4.32 (d, J=4.8 Hz, 2H), 3.07 (s, 6H), 2.43 (s, 3H).

Example 2: Synthesis of 4-(7-(2-Fluoroethoxy)quinoxalin-2-yl)-N,N-dimethylaniline (E3; Non-Radioactive Fluorine-Labeled Form, Compound 5)

The non-radioactive fluorine-labeled form of [$^{18}$F]E3 was synthesized as follows according to the scheme 2 shown in FIG. 1.

Step 2:

In N,N-dimethylformamide (10 mL) was dissolved 3-(4-(dimethylamino)phenyl)quinoxalin-6-ol (56.0 mg, 0.211 mmol) synthesized in the step 1-6 of Example 1, to which potassium carbonate (58.4 mg, 0.422 mmol) was then added. The resultant was heat-stirred at 105°C for 0.5 hour, and 2-fluoroethyl-4-methylbenzenesulfonate (50 μL) was added thereto, which was then heat-stirred at 105°C for 1.5 hours. 2-Fluoroethyl-4-methylbenzenesulfonate (30 μL) was added thereto, which was then heat-stirred at 105°C for 3 hours. In addition, 2-fluoroethyl-4-methylbenzenesulfonate (40 μL) was added thereto, which was then heat-stirred at 105°C for 0.5 hour. The reaction solution was cooled down to room temperature, and separation and extraction were performed with ethyl acetate (60 mL × 2). The organic layer was dehydrated and dried with magnesium sulfate, and after distilling off the solvent under reduced pressure, the resultant residue was purified by silica-gel chromatography (ethyl acetate/hexane = 1/2 (volume ratio)) to provide 4-(7-(2-fluoroethoxy)quinoxalin-2-yl)-N,N-dimethylaniline (hereinafter, also referred to as "E3" or "compound 5") as the desired product (yield: 33.4 mg (50.8%)).

$^1$H-NMR (400 MHz, deuterated chloroform) δ 9.14 (s, 1H), 8.11 (d, J=9.2 Hz, 2H), 7.95 (d, J=9.6 Hz, 1H), 7.37-7.34 (m, 2H), 6.84 (d, J=9.6 Hz, 2H), 4.80 (m, 2H), 4.20 (m, 2H), 3.08 (s, 6H).

Example 3: Synthesis of 2-((2-(4-(dimethylamino)phenyl)quinoxalin-6-yl)oxy)ethyl-4-methylbenzenesulfonate (28)

Compound 4 as a labeling precursor for [$^{18}$F]D3 was synthesized as follows according to the scheme 3 shown in FIG. 2.

Step 3-1: Synthesis of 2-chloroquinoxalin-6-ol (25)

Aluminum chloride (160.0 mg, 1.2 mmol) was slowly added to toluene (4 mL) in an environment cooled at 0°C, to which 2-chloro-6-methoxyquinoxaline (97.3 mg, 0.5 mmol) was further added, followed by heat-stirring the resultant at 80°C for 4 hours. The reaction was stopped by adding water in an environment cooled at 0°C, and after separation and extraction with ethyl acetate (60 mL × 2), the organic layer was dehydrated and dried with sodium sulfate. The solvent was distilled off under reduced pressure, and the resultant residue was purified by silica-gel chromatography (chloroform/methanol = 30/1 (volume ratio)) to provide (25) in the scheme 3 (yield: 59.3 mg (65.7%)).
$^1$H-NMR (400 MHz, deuterated methanol) δ 8.71 (s, 1H), 7.84 (d, J=9.2 Hz, 1H), 7.44 (dd, J=9.2, 2.8 Hz, 1H), 7.32 (d, J=2.8 Hz, 1H).

Step 3-2: Synthesis of 6-(2-((tert-Butyldimethylsilyl)oxy)ethoxy)-2-chloroquinoxaline (26)

In N,N-dimethylformamide (7 mL) was dissolved 2-chloroquinoxalin-6-ol (59.3 mg, 0.328 mmol) synthesized in the step 3-1, to which potassium carbonate (90.8 mg, 0.657 mmol) was then added. The resultant was heat-stirred at 80°C for 0.5 hour, and (2-bromoethoxy)-tert-butyldimethylsilane (140 μL) was added thereto, which was then heat-stirred at 80°C for further 2.5 hours. The reaction solution was cooled down to room temperature, and after separation and extraction with ethyl acetate (60 mL × 2), the organic layer was dehydrated and dried with magnesium sulfate. The solvent was distilled off under reduced pressure, and the resultant residue was purified by silica-gel chromatography (ethyl acetate/hexane = 1/10 (volume ratio)) to provide (26) in the scheme 3 (yield: 101.5 mg (91.3%)).
$^1$H-NMR (400 MHz, deuterated chloroform) δ 8.71 (s, 1H), 7.90 (d, J=9.2 Hz, 1H), 7.47 (dd, J=9.2, 2.8 Hz, 1H), 7.39 (d, J=2.8 Hz, 1H), 4.21 (t, J=4.8 Hz, 2H), 4.06 (t, J=4.8 Hz, 2H), 0.92 (s, 9H), 0.12 (s, 6H).

Step 3-3: Synthesis of 4-(6-(2-((tert-Butyldimethylsilyl)oxy)ethoxy)quinoxalin-2-yl)-N,N-dimethylaniline (27)

In toluene (2.5 mL) was dissolved 2-bromo-1-(4-(dimethylamino)phenyl)ethanone (101.5 mg, 0.229 mmol), to which ethanol (350 μL) was then added. In addition, thereto were added N,N-dimethyl-4-(4,4,5,5-tetramethyl-1,3,2-dioxaboran-2-yl)aniline (81.3 mg, 0.329 mmol), tetrakis(triphenylphosphine)palladium (10.4 mg, 0.00897 mmol), and a 2 mol/L potassium carbonate aqueous solution (329 μL) in that order, which was then reflux-stirred at 110°C for 3 hours. The reaction solution was cooled down to room temperature, and after separation and extraction with ethyl acetate (60 mL × 2), the organic layer was dehydrated and dried with magnesium sulfate. The solvent was distilled off under reduced pressure, and the resultant residue was purified by silica-gel chromatography (ethyl acetate/hexane = 1/3 (volume ratio)) to provide (27) in the scheme 3 (yield: 127.0 mg (100%)).
$^1$H-NMR (400 MHz, deuterated chloroform) δ 9.19 (s, 1H), 8.08 (d, J=8.8 Hz, 2H), 7.96 (d, J=9.2 Hz, 1H), 7.40 (dd, J=9.2, 2.8 Hz, 1H), 7.35 (d, J=2.8 Hz, 1H), 6.84 (d, J=8.8 Hz, 2H), 4.21 (t, J=4.8 Hz, 2H), 4.06 (t, J=4.8 Hz, 2H), 3.06 (s, 6H), 0.93 (s, 9H), 0.13 (s, 6H).

Step 3-4: Synthesis of 2-((2-(4-(Dimethylamino)phenyl) quinoxalin-6-yl)oxy)ethyl-4-methylbenzenesulfonate (Compound 4)

In tetrahydrofuran (5 mL) was dissolved 4-(6-(2-((tert-Butyldimethylsilyl)oxy)ethoxy)quinoxalin-2-yl)-N,N-dimethylaniline (127.0 mg, 0.300 mmol) synthesized in the step 3-3, to which tetra-n-butylammonium fluoride (1 mol/L, tetrahydrofuran solution) (360 μL, 0.360 mmol) was then slowly added in an environment cooled at 0°C. The resultant was stirred at room temperature for 1 hour, and after separation and extraction with ethyl acetate (60 mL × 2), the solvent was distilled off under reduced pressure. The resultant residue was dissolved in pyridine (5 mL), to which were then added para-toluenesulfonyl chloride (286.0 mg, 1.50 mmol) and N,N-dimethyl-4-aminopyridine (3.67 mg, 0.030 mmol), followed by stirring the resultant at room temperature for 6 hours. The solvent was distilled off under reduced pressure, and separation and extraction were performed with ethyl acetate (60 mL × 2). After dehydrating and drying the organic layer with magnesium sulfate, the solvent was distilled off under reduced pressure, and the resultant residue was purified by silica-gel chromatography (ethyl acetate/hexane = 1/1 (volume ratio)) to provide 2-((2-(4-(dimethylamino)phenyl)quinoxalin-6-yl)oxy)ethyl-4-methylbenzenesulfonate (hereinafter, also referred to as "compound 4") as the desired product (yield: 64.0 mg (46.0%)).
$^1$H-NMR (400 MHz, deuterated chloroform) $\delta$ 9.19 (s, 1H), 8.08 (d, J=8.8 Hz, 2H), 7.94 (d, J=9.2 Hz, 1H), 7.84 (d, J=8.8 Hz, 2H), 7.33 (d, J=8.0 Hz, 2H), 7.26-7.21 (m, 2H), 6.84 (d, J=8.8 Hz, 2H), 4.49-4.46 (m, 2H), 4.31-4.29 (m, 2H), 3.07 (s, 6H), 2.43 (s, 3H).

Example 4: Synthesis of 4-(6-(2-fluoroethoxy)quinoxalin-2-yl)-N,N-dimethylaniline (D3; Non-Radioactive Fluorine-Labeled Form, Compound 6)

The non-radioactive fluorine-labeled form of D3 was synthesized as follows according to the scheme 1 shown in FIG. 1 and the scheme 4 shown in FIG. 2.

Step 4-1: Synthesis of 4-(6-((tert-Butyldimethylsilyl)oxy)quinoxalin-2-yl)-N,N-dimethylaniline (5)

In dimethyl sulfoxide (10 mL) was dissolved 4-((tert-butyldimethylsilyl)oxy)benzene-1,2-diamine (476.3 mg, 2 mmol) synthesized in the step 1-4, to which was then added 2-bromo-1-(4-(dimethylamino)phenyl)ethanone (482.0 mg, 2 mmol) synthesized in the step 1-2. The resultant was stirred at room temperature for 4 hours, and after separation and extraction with ethyl acetate (100 mL $\times$ 2), the organic layer was dehydrated and dried with magnesium sulfate. The solvent was distilled off under reduced pressure, and the resultant residue was purified by silica-gel chromatography (ethyl acetate/hexane = 1/3 (volume ratio)). Here, a fraction with Rf = 0.50 was separated to provide (5) in the scheme 1 (yield: 46.5 mg (6.1%)).
$^1$H-NMR (400 MHz, deuterated chloroform) $\delta$ 9.19 (s, 1H), 8.08 (d, J=8.8 Hz, 2H), 7.96 (d, J=9.2 Hz, 1H), 7.40 (d, J=2.4 Hz, 1H), 7.32 (dd, J=9.2, 2.4 Hz, 1H), 6.85 (d, J=9.2 Hz, 2H), 3.07 (s, 6H), 1.03 (s, 9H), 0.30 (s, 6H).

Step 4-2: Synthesis of 4-(6-(2-Fluoroethoxy)quinoxalin-2-yl)-N,N-dimethylaniline (D3, Compound 6)

In tetrahydrofuran (10 mL) was dissolved 4-(6-((tert-butyldimethylsilyl)oxy)quinoxalin-2-yl)-N,N-dimethylaniline (87.2 mg, 0.230 mmol) synthesized in the step 4-1, to which was then slowly added tetra-n-butylammonium fluoride (1 mol/L, tetrahydrofuran solution) (276 $\mu$L, 0.276 mmol) under conditions cooled at 0°C. The resultant was stirred at room temperature for 0.5 hour; after separation and extraction with ethyl acetate (60 mL $\times$ 2), the organic layer was dehydrated and dried with magnesium sulfate; and the solvent was distilled off under reduced pressure. The resultant residue was dissolved in N,N-dimethylformamide (10 mL), to which potassium carbonate (63.6 mg, 0.460 mmol) was then added. The resultant was heat-stirred at 105°C for 0.7 hour, and 2-fluoroethyl-4-methylbenzenesulfonate (100 $\mu$L) was added thereto, which was then heat-stirred at 105°C for further 1 hour. In addition, thereto were added potassium carbonate (63.6 mg, 0.460 mmol) and 2-fluoroethyl-4-methylbenzenesulfonate (200 $\mu$L), which was then heat-stirred at 100°C overnight. Cesium carbonate (150 mg, 0.460 mmol) was added thereto, which was then heat-stirred at 100°C for 0.5 hour. In addition, 2-fluoroethyl-4-methylbenzenesulfonate (200 $\mu$L) was added thereto, which was then heat-stirred at 100°C overnight. The reaction solution was cooled down to room temperature, and separation and extraction were performed with ethyl acetate (60 mL $\times$ 2). After dehydrating and drying the organic layer with magnesium sulfate and distilling off the solvent under reduced pressure, the resultant residue was purified by silica-gel chromatography (chloroform/methanol = 40/1 (volume ratio)) to provide 4-(6-(2-fluoroethoxy)quinoxalin-2-yl)-N,N-dimethylaniline (hereinafter, also referred to as "D3" or "compound 6") as the desired product (yield: 65.5 mg (91.5%)).
$^1$H-NMR (400 MHz, deuterated chloroform) $\delta$ 9.20 (s, 1H), 8.10 (d, J=8.8 Hz, 2H), 7.99 (d, J=9.2 Hz, 1H), 7.44 (dd, J=9.2, 2.8 Hz, 1H), 7.35 (d, J=2.4 Hz, 1H), 4.92-4.78 (m, 2H), 4.43-4.34 (m, 2H), 3.07 (s, 6H).

Comparative Example 1: Synthesis of 4-(7-(2-Fluoroethoxy)quinoxalin-2-yl)aniline (E1; Non-Radioactive Fluorine-Labeled Form, Compound 11)

The non-radioactive fluorine-labeled form of [$^{18}$F]E1 was synthesized as follows according to the scheme 5 shown in FIG. 3.

Step 5-1: Synthesis of 7-((tert-Butyldimethylsilyl)oxy)-2-(4-nitrophenyl)quinoxaline (12)

In dimethyl sulfoxide (20 mL) was dissolved 4-((tert-butyldimethylsilyl)oxy)benzene-1,2-diamine (952.6 mg, 4 mmol) synthesized in the step 1-4, to which was then added 2-bromo-4'-nitroacetophenone (976.2 mg, 4 mmol). The resultant was stirred at room temperature for 2 hours, and after separation and extraction with ethyl acetate (200 mL $\times$ 2), the organic layer was dehydrated and dried with magnesium sulfate. The solvent was distilled off under reduced pressure, and the resultant residue was purified by silica-gel chromatography (ethyl acetate/hexane = 1/3 (volume ratio))

to provide (12) in the scheme 5 (yield: 567.1 mg (37.2%)).
$^{1}$H-NMR (400 MHz, deuterated chloroform) δ 9.22 (s, 1H), 8.43-8.36 (m, 4H), 8.03 (d, J=8.8 Hz, 1H), 7.49 (d, J=2.4, 1H), 7.40 (dd, J=9.2, 2.8 Hz, 1H), 1.04 (s, 9H), 0.34 (s, 6H).

Step 5-2: Synthesis of 3-(4-Nitrophenyl)quinoxalin-6-ol (13)

In tetrahydrofuran (15 mL) was dissolved 7-((tert-butyldimethylsilyl)oxy)-2-(4-nitrophenyl)quinoxaline (398.8 mg, 1.05 mmol) synthesized in the step 5-1, to which was then slowly added tetra-n-butylammonium fluoride (1 mol/L, tetrahydrofuran solution) (1.26 mL, 1.26 mmol) in an environment cooled at 0°C. The resultant was stirred at room temperature for 2 hours, and after separation and extraction with ethyl acetate (60 mL × 2), the organic layer was dehydrated and dried with magnesium sulfate. The solvent was distilled off under reduced pressure, and the resultant residue was purified by silica-gel chromatography (ethyl acetate/hexane = 1/1 (volume ratio)) to provide (13) in the scheme 5 (yield: 150.1 mg (53.7%)).
$^{1}$H-NMR (400 MHz, DMSO-d6) δ 10.7 (s, 1H), 9.44 (s, 1H), 8.57 (d, J=9.2 Hz, 2H), 8.41 (d, J=8.8 Hz, 2H), 8.00 (d, J=9.2 Hz, 1H), 7.46 (dd, J=9.2, 2.8 Hz, 1H), 7.36 (d, J=2.4 Hz, 1H).

Step 5-3: Synthesis of 4-(7-(2-Fluoroethoxy)quinoxalin-2-yl)aniline (E1, Compound 11)

In N,N-dimethylformamide (10 mL) was dissolved 3-(4-nitrophenyl)quinoxalin-6-ol (151.4 mg, 0.576 mmol) synthesized in the step 5-2, to which potassium carbonate (159.2 mg, 1.152 mmol) was then added. The resultant was heat-stirred at 105°C for 0.5 hour, and 2-fluoroethyl-4-methylbenzenesulfonate (300 μL) was added thereto, which was then heat-stirred at 105°C for further 4.5 hours. The reaction solution was cooled down to room temperature, and separation and extraction were performed with ethyl acetate (60 mL × 2). The organic layer was dehydrated and dried with magnesium sulfate, and the solvent was distilled off under reduced pressure. The resultant residue was dissolved in a mixed solvent of methanol (40 mL) and dichloromethane (30 mL), to which palladium carbon (100 mg) was then added. The resultant was stirred at room temperature for 0.7 hour under hydrogen filling. The resultant was filtered with celite, and the filtrate was distilled off under reduced pressure. The residue was purified by silica-gel chromatography (acetonitrile/hexane = 1/1 (volume ratio)) to provide 4-(7-(2-fluoroethoxy)quinoxalin-2-yl)aniline (hereinafter, also referred to as "E1" or "compound 11") as the desired product (yield: 90.4 mg (55.4%)).
$^{1}$H-NMR (400 MHz, deuterated chloroform) δ 9.12 (s, 1H), 8.04 (d, J=8.4 Hz, 2H), 7.96 (d, J=9.2 Hz, 1H), 7.40-7.37 (m, 2H), 6.83 (d, J=8.8 Hz, 2H), 4.93-4.79 (m, 2H), 4.44-4.35 (m, 2H).

Comparative Example 2: Synthesis of 4-(7-(2-Fluoroethoxy)quinoxalin-2-yl)-N-methylaniline (E2; Non-Radioactive Fluorine-Labeled Form, Compound 12)

Step 6:

The non-radioactive fluorine-labeled form of [$^{18}$F]E2 was synthesized as follows according to the scheme 5 shown in FIG. 3.
In methanol (35 mL) was dissolved 4-(7-(2-fluoroethoxy)quinoxalin-2-yl)aniline (90.4 mg, 0.319 mmol) synthesized in the step 5-3 of Comparative Example 1, to which was then added paraformaldehyde (51.7 mg, 1.72 mmol). Sodium methoxide (256 μL, 1.28 mmol) was slowly added thereto in an environment cooled at 0°C. This was reflux-stirred at 75°C for 1.5 hours. Thereafter, the resultant was cooled down to room temperature, and sodium boron hydride (60.5 mg, 1.60 mmol) was added thereto. The resultant was reflux-stirred at 75°C for 3 hours. This was neutralized with saturated sodium hydrogencarbonate, and after distilling off the solvent under reduced pressure, separation and extraction were performed with ethyl acetate (60 mL × 2). The organic layer was dehydrated and dried with magnesium sulfate; the solvent was distilled off under reduced pressure; and the resultant residue was purified by silica-gel chromatography (ethyl acetate/hexane = 1/1 (volume ratio)) to provide 4-(7-(2-fluoroethoxy)quinoxalin-2-yl)-N-methylaniline (hereinafter, also referred as "E2" or "compound 12") as the desired product (yield: 19.4 mg (20.5%)).
$^{1}$H-NMR (400 MHz, deuterated chloroform) δ 9.13 (s, 1H), 8.07 (d, J=8.8 Hz, 2H), 7.95 (d, J=9.2 Hz, 1H), 7.38-7.35 (m, 2H), 6.74 (d, J=8.8 Hz, 2H), 4.93-4.79 (m, 2H), 4.44-4.35 (m, 2H), 2.94 (s, 3H).

Comparative Example 3: Synthesis of 2-((3-(4-((Di-tert-butoxycarbonyl)amino)phenyl)quinoxalin-6-yl)oxy)ethyl-4-methylbenzenesulfonate (Compound 9)

Compound 9 as a labeling precursor for [$^{18}$F]E1 was synthesized as follows according to the scheme 6 shown in FIG. 4.

Step 7-1: Synthesis of 7-(2-((tert-Butyldimethylsilyl)oxy)ethoxy)-2-(4-nitrophenyl)-quinoxaline (16)

In N,N-dimethylformamide (15 mL) was dissolved 3-(4-nitrophenyl)quinoxalin-6-ol (150.1 mg, 0.562 mmol) synthesized in the step 5-2 of Comparative Example 1, to which potassium carbonate (155.3 mg, 1.12 mmol) was then added. The resultant was heat-stirred at 80°C for 1 hour, and (2-bromoethoxy)-tert-butyldimethylsilane (250 μL) was added thereto, which was then heat-stirred at 80°C overnight. The reaction solution was cooled down to room temperature, and after separation and extraction with ethyl acetate (60 mL × 2), the organic layer was dehydrated and dried with magnesium sulfate. The solvent was distilled off under reduced pressure, and the resultant residue was purified by silica-gel chromatography (ethyl acetate/hexane = 1/3 (volume ratio)) to provide (16) in the scheme 6 (yield: 203.8 mg (85.3%)).
$^1$H-NMR (400 MHz, deuterated chloroform) δ 9.22 (s, 1H), 8.43-8.36 (m, 4H), 8.04 (d, J=9.2 Hz, 1H), 7.51-7.46 (m, 2H), 7.26 (t, J=4.8 Hz, 2H), 4.09 (t, J=4.8 Hz, 2H), 0.93 (s, 9H), 0.14 (s, 6H).

Step 7-2: Synthesis of 4-(7-(2-((tert-Butyldimethylsilyl)oxy)ethoxy)quinoxalin-2-yl)aniline (17)

In a mixed solvent of methanol (10 mL) and dichloromethane (5 mL) was dissolved 7-(2-((tert-butyldimethylsilyl)oxy)ethoxy)-2-(4-nitrophenyl)-quinoxaline (107.5 mg, 0.253 mmol) synthesized in the step 7-1, to which palladium carbon (100 mg) was then added. The resultant was stirred at room temperature for 0.5 hour under hydrogen filling. The resultant was filtered with celite, and the filtrate was distilled off under reduced pressure. The residue was purified by silica-gel chromatography (chloroform/methanol = 40/1 (volume ratio)) to provide (17) in the scheme 6 (yield: 90.5 mg (90.6%)).
$^1$H-NMR (400 MHz, deuterated chloroform) δ 9.09 (s, 1H), 8.03 (d, J=8.8 Hz, 2H), 7.93 (d, J=9.2 Hz, 1H), 7.38-7.32 (m, 2H), 6.83 (d, J=8.8 Hz, 2H), 4.22 (t, J=4.8 Hz, 2H), 4.07 (t, J=4.8 Hz, 2H), 3.95 (s, 2H), 0.93 (s, 9H), 0.13 (s, 6H).

Step 7-3: Synthesis of 7-(2-((tert-Butyldimethylsilyl)oxy)ethoxy)-2-(para-di(tert-butoxycarbonyl)aminophenyl)quinoxaline (18)

In tetrahydrofuran (10 mL) was dissolved 4-(7-(2-((tert-butyl-dimethylsilyl)oxy)ethoxy)quinoxalin-2-yl)aniline (90.5 mg, 0.229 mmol) synthesized in the step 7-2, to which were then added di-tert-butyl-di-carbamate (499.8 mg, 2.29 mmol), N,N-dimethyl-4-aminopyridine (catalytic amount), and triethylamine (38.2 μL), followed by reflux-stirring the resultant at 80°C for 1.5 hours. The reaction solution was cooled down to room temperature, and after separation and extraction with ethyl acetate (60 mL × 2), the organic layer was dehydrated and dried with magnesium sulfate. The solvent was distilled off under reduced pressure, and the resultant residue was purified by silica-gel chromatography (ethyl acetate/hexane = 1/3 (volume ratio)) to provide (18) in the scheme 6 (yield: 64.5 mg (47.3%)).
$^1$H-NMR (400 MHz, deuterated chloroform) δ 9.18 (s, 1H), 8.19 (d, J=8.8 Hz, 2H), 7.99 (d, J=9.6 Hz, 1H), 7.44-7.41 (m, 2H), 7.33 (d, J=8.8 Hz, 2H), 4.24 (t, J=4.8 Hz, 2H), 4.08 (t, J=4.8 Hz, 2H), 1.44 (s, 18H), 0.93 (s, 9H), 0.13 (s, 6H).

Step 7-4: Synthesis of 7-(2-Hydroxyethoxy)-2-(p-di-(tert-butoxycarbonyl)aminophenyl)quinoxaline (19)

In tetrahydrofuran (5 mL) was dissolved 7-(2-((tert-butyldimethylsilyl)oxy)ethoxy)-2-(para-di(tert-butoxycarbonyl)aminophenyl)quinoxaline (64.5 mg, 0.108 mmol) synthesized in the step 7-3, to which was then slowly added tetra-n-butylammonium fluoride (1 mol/L, tetrahydrofuran solution) (130 μL, 0.130 mmol) in an environment cooled at 0°C. The resultant was stirred at room temperature overnight, and after separation and extraction with ethyl acetate (60 mL × 2), the organic layer was dehydrated and dried with magnesium sulfate. The solvent was distilled off under reduced pressure, and the resultant residue was purified by silica-gel chromatography (chloroform/methanol = 20/1 (volume ratio)) to provide (19) in the scheme 6 (yield: 54.0 mg (100%)).
$^1$H-NMR (400 MHz, deuterated chloroform) δ 9.20 (s, 1H), 8.20 (d, J=8.8 Hz, 2H), 8.02 (d, J=9.6 Hz, 1H), 7.46-7.43 (m, 2H), 7.34 (d, J=8.8 Hz, 2H), 4.30 (t, J=4.8 Hz, 2H), 4.09 (t, J=4.8 Hz, 2H), 1.44 (s, 18H).

Step 7-5: Synthesis of 2-((3-(4-((di-tert-butoxycarbonyl)amino)phenyl)quinoxalin-6-yl)oxy)-ethyl-4-methylbenzenesulfonate (Compound 9)

In pyridine (7 mL) was dissolved 7-(2-hydroxyethoxy)-2-(p-di-(tert-butoxycarbonyl)aminophenyl)quinoxaline (54.0 mg, 0.112 mmol) synthesized in the step 7-4, to which were then added N,N-dimethyl-4-aminopyridine (catalytic amount) and para-toluenesulfonyl chloride (107.0 mg, 0.561 mmol), followed by stirring the resultant at room temperature overnight. The solvent was distilled off under reduced pressure, and separation and extraction were performed with chloroform (60 mL × 2). After dehydrating and drying the organic layer with magnesium sulfate, the solvent was distilled off under reduced pressure. The resultant residue was purified by silica-gel chromatography (ethyl acetate/hexane = 1/1 (volume ratio)) to provide 2-((3-(4-((di-tert-butoxycarbonyl)amino)phenyl)quinoxalin-6-yl)oxy)-ethyl-4-methylbenzenesulfonate (hereinafter, also referred to as "compound 9") as the desired product (yield: 36.0 mg (50.6%)).
$^1$H-NMR (400 MHz, deuterated chloroform) δ 9.19 (s, 1H), 8.19 (d, J=8.8 Hz, 2H), 7.98 (d, J=9.2 Hz, 1H), 7.85 (d, J=8.4 Hz, 2H), 7.35-7.27 (m, 6H), 4.50-4.48 (m, 2H), 4.35-4.33 (m, 2H), 2.44 (s, 2H), 1.44 (s, 18H).

Comparative Example 4: Synthesis of 2-((3-(4-((tert-Butoxycarbonyl)(methyl)amino)phenyl)quinoxalin-6-yl)oxy)ethyl-4-methylbenzenesulfonate (Compound 10)

Compound 10 as a labeling precursor for [$^{18}$F]E2 was synthesized as follows according to the scheme 7 shown in FIG. 5.

Step 8-1: Synthesis of 4-(7-(2-((tert-Butyldimethylsilyl)oxy)ethoxy)quinoxalin-2-yl)-N-methylaniline (21)

In methanol (40 mL) was dissolved 4-(7-(2-((tert-butyl-dimethylsilyl)oxy)ethoxy)quinoxalin-2-yl)aniline (215.1 mg, 0.544 mmol) synthesized in the step 7-2 of Comparative Example 3, to which was then added paraformaldehyde (88.3 mg, 2.94 mmol). Sodium methoxide (436 μL, 2.18 mmol) was slowly added thereto at 0°C, which was then reflux-stirred at 75°C for 4.5 hours. Then, the resultant was cooled down to room temperature, and sodium boron hydride (102.9 mg, 2.72 mmol) was added thereto, which was then reflux-stirred at 75°C for 1 hour. This was neutralized with saturated sodium hydrogencarbonate, and after distilling off the solvent under reduced pressure, separation and extraction were performed with ethyl acetate (60 mL × 2). After dehydrating and drying the organic layer with magnesium sulfate, the solvent was distilled off under reduced pressure, and the resultant residue was purified by silica-gel chromatography (ethyl acetate/hexane = 1/1 (volume ratio)) to provide (21) shown in the scheme 7 (yield: 131.6 mg (59.1%)).
$^1$H-NMR (400 MHz, deuterated chloroform) δ 9.10 (s, 1H), 8.07 (d, J=8.8 Hz, 2H), 7.92 (d, J=9.2 Hz, 1H), 7.37 (d, J=2.4 Hz, 1H), 7.32 (d, J=9.2 Hz, 1H), 6.74 (d, J=8.8 Hz, 2H), 4.22 (t, J=4.8 Hz, 2H), 4.07 (t, J=4.8 Hz, 2H), 2.93 (s, 3H), 0.93 (s, 9H), 0.13 (s, 6H).

Step 8-2: Synthesis of tert-Butyl(4-(7-(2-((tert-butyldimethylsilyl)oxy)ethoxy)quinoxalin-2-yl)phenyl)(methyl)carbamate (22)

In tetrahydrofuran (10 mL) was dissolved 4-(7-(2-((tert-butyldimethylsilyl)oxy)ethoxy)quinoxalin-2-yl)-N-methylaniline (131.6 mg, 0.322 mmol) synthesized in the step 8-1, to which was then added di-tert-butyldicarbonate (140.6 mg, 0.644 mmol), followed by reflux-stirring the resultant at 80°C for 2 hours. Thereto were added N,N-dimethyl-4-aminopyridine (catalytic amount) and triethylamine (54 μL), which was reflux-stirred at 80°C for 5 hours. The reaction solution was cooled down to room temperature, and after separation and extraction with ethyl acetate (60 mL × 2), the organic layer was dehydrated and dried with magnesium sulfate. The solvent was distilled off under reduced pressure, and the resultant residue was purified by silica-gel chromatography (ethyl acetate/hexane = 1/2 (volume ratio)) to provide (22) shown in the scheme 7 (yield: 133.1 mg (81.2%)).
$^1$H-NMR (400 MHz, deuterated chloroform) δ 9.15 (s, 1H), 8.14 (d, J=8.8 Hz, 2H), 7.98 (d, J=9.2 Hz, 1H), 7.46-7.39 (m, 4H), 4.24 (t, J=4.8 Hz, 2H), 4.08 (t, J=4.8 Hz, 2H), 3.34 (s, 3H), 1.49 (s, 9H), 0.93 (s, 9H), 0.13 (s, 6H).

Step 8-3: Synthesis of tert-Butyl(4-(7-(2-hydroxyethoxy)quinoxalin-2-yl)phenyl)(methyl)-carbamate (23)

In tetrahydrofuran (7 mL) was dissolved tert-butyl(4-(7-(2-((tert-butyldimethyl)oxy)ethoxy)quinoxalin-2-yl)phenyl)(methyl)carbamate (133.1 mg, 0.261 mmol) synthesized in the step 8-2, to which was then slowly added tetra-n-butylammonium fluoride (1 mol/L, tetrahydrofuran solution) (314 μL, 0.314 mmol) in an environment cooled at 0°C. The resultant was stirred at room temperature for 2 hours, and after separation and extraction with ethyl acetate (60 mL × 2), the organic layer was dehydrated and dried with magnesium sulfate. The solvent was distilled off under reduced pressure, and the resultant residue was purified by silica-gel chromatography (chloroform/methanol = 20/1 (volume ratio)) to provide (23) shown in the scheme 7 (yield: 104.1 mg (100%)).
$^1$H-NMR (400 MHz, deuterated chloroform) δ 9.17 (s, 1H), 8.14 (d, J=8.8 Hz, 2H), 8.00 (d, J=9.2 Hz, 1H), 7.46-7.40 (m, 4H), 4.30-4.28 (m, 2H), 4.09-4.08 (m, 2H), 3.34 (s, 3H), 2.08 (s, 1H), 1.49 (s, 9H).

Step 8-4: Synthesis of 2-((3-(4-((tert-Butoxycarbonyl)(methyl)amino)phenyl)quinoxalin-6-yl)oxy)ethyl-4-methylbenzenesulfonate (Compound 10)

In pyridine (10 mL) was dissolved tert-butyl(4-(7-(2-hydroxyethoxy)quinoxalin-2-yl)phenyl)(methyl)-carbamate (104.1 mg, 0.263 mmol) synthesized in the step 8-3, to which was then added para-toluenesulfonyl chloride (107.0 mg, 0.561 mmol), followed by stirring the resultant at room temperature for 1 hour. Thereto was added N,N-dimethyl-4-aminopyridine (catalytic amount), which was then stirred at room temperature overnight. The solvent was distilled off

under reduced pressure, and separation and extraction were performed with chloroform (60 mL × 2). After dehydrating and drying the organic layer with magnesium sulfate, the solvent was distilled off under reduced pressure. The resultant residue was purified by silica-gel chromatography (ethyl acetate/hexane = 1/1 (volume ratio)) to provide 2-((3-(4-((tert-butoxycarbonyl)(methyl)amino)phenyl)quinoxalin-6-yl)oxy)ethyl-4-methylbenzenesulfonate (hereinafter, also referred to as "compound 10") as the desired product (yield: 92.2 mg (63.9%)).
$^1$H-NMR (400 MHz, deuterated chloroform) δ 9.17 (s, 1H), 8.14 (d, J=8.8 Hz, 2H), 7.97 (d, J=9.2 Hz, 1H), 7.85 (d, J=8.4 Hz, 2H), 7.45 (d, J=8.8 Hz, 2H), 7.35-7.25 (m, 4H), 4.50-4.48 (m, 2H), 4.35-4.33 (m, 2H), 3.34 (s, 3H), 2.44 (s, 3H), 1.49 (s, 9H).

Example 5: [$^{125}$I]IMPY Binding Inhibition Experiment

To evaluate the affinity of each compound for $A\beta_{1-42}$ aggregates, an experiment was performed of inhibition of binding of [$^{125}$I]6-iodo-2-(4'-dimethylamino)phenyl-imidazo[1,2-a]pyridine ([$^{125}$I]IMPY) known to selectively bind to Aβ plaques, to $A\beta_{1-42}$ aggregates. IMPY (non-radioactive iodine-labeled form) and [$^{125}$I]IMPY were synthesized according to a method as described in literature (Zhi-Ping Zhuang et al., J. Med. Chem, 2003, 46, p.237-243) (radiochemical purity: 99% or more, 0.1 to 0.5 MBq).
$A\beta_{1-42}$ aggregates (Peptide Institute, Inc, Osaka) were dissolved in 0.1 mol/L phosphate-buffered saline (hereinafter referred to as PBS) to 0.25 mg/mL and incubated at 37°C for 42 hours to prepare an $A\beta_{1-42}$ aggregate PBS solution. This was further diluted 1:100 with PBS to 2.5 μg/mL and used for an assay.

Mixed were 50 μL of each of ethanol solutions of IMPY (non-radioactive iodine-labeled form), E1 to 3, and D3 (final concentrations: 12 concentrations between 0.5 pmol/L and 25 μmol/L), 50 μL of an ethanol solution of [$^{125}$I]IMPY (final concentration: 0.025 nmol/L, 2.03 MBq), 50 μL of an $A\beta_{1-42}$ aggregate solution (final concentration: 0.125 μg/mL), and 850 μL of a 10 volume% ethanol aqueous solution, which was then incubated at room temperature for 3 hours. The mixture was filtered through Whatman (R) GF/B filter, and a γ counter was used to measure the radioactivity of the filter. The measured radioactivity values were used to prepare an inhibition curve to calculate $IC_{50}$. In addition, the following mathematical expression (1):

$$Ki = IC_{50} / (1 + [L] / Kd) \qquad (1)$$

was used to calculate an inhibition constant (Ki). In the expression (1), [L] is the concentration of [$^{125}$I]IMPY used in the assay and Kd is 4.2 nmol/L as the dissociation constant of IMPY.

The Ki value of each compound is shown in Table 2. E3 and D3 were each confirmed to have a sufficiently high binding property to $A\beta_{1-42}$ aggregates. Particularly, D3 also exhibited a high binding property to $A\beta_{1-42}$ aggregates compared to IMPY of interest. On the other hand, E1 and E2 were not each confirmed to have a sufficient binding property to $A\beta_{1-42}$ aggregates.

Table 2 Ki Value of Each Compound (Each value is in nmol/L and values in parentheses show SE)

| IMPY | E3 | D3 | E1 | E2 |
|---|---|---|---|---|
| 7.21 (1.23) | 111 (13.2) | 0.947 (0.212) | 1180 (370) | 758 (84) |

Example 6: Synthesis of $^{18}$F-Labeled Compound

$H_2{}^{18}O$ containing [$^{18}$F]fluoride ion was passed through Sep-Pak Light QMA cartridge (trade name, from Nihon Waters K.K.) to adsorb and collect [$^{18}$F]fluoride ion. Then, a potassium carbonate aqueous solution (66 mmol/L, 0.3 mL) was passed through the column to elute $^{18}$F, and 10 mg of Kryptofix 222 (trade name, from Merck & Co., Inc.) was dissolved therein. The solvent was distilled off at 120°C in a nitrogen atmosphere; dehydrated acetonitrile (0.3 mL) was further added to the residue; and the solvent was distilled off at 120°C in a nitrogen atmosphere (repeated 3 times).

Each of the compounds 3, 4, 9, and 10 (1.0 mg each) as labeling precursors was dissolved in dehydrated acetonitrile (300 μL), which was added to an reaction vessel containing the above-described residue containing $^{18}$F (3.7 to 5.6 GBq). The reaction solution was heated at 100°C for 10 minutes. In the labeling reaction of each of the compounds 9 and 10, 1 mol/L hydrochloric acid (450 μL) was further added thereto, which was then heated at 100°C for further 10 minutes, cooled down to room temperature, and then neutralized by adding saturated sodium hydrogencarbonate (500 μL); after separation and extraction with ethyl acetate (1 mL), the solvent was distilled off under reduced pressure, and the resultant residue was dissolved in dehydrated acetonitrile (300 μL). Each reaction solution was passed through a

filter and then purified using HPLC under the following conditions to provide a $^{18}F$-labeled compound. $^{18}F$-labeled compounds for the compounds 3, 4, 9, and 10 are referred to as [$^{18}F$]E3 (compound 1), [$^{18}F$]D3 (compound 2), [$^{18}F$]E1 (compound 7), and [$^{18}F$]E2 (compound 8), respectively.

[$^{18}F$]E3 and [$^{18}F$]D3:

Column: Cosmosil (R) C18 column (from Nacalai Tesque, Inc)
Mobile phase: acetonitrile/water (5/5)
Flow rate: 1.0 mL/minute

[$^{18}F$]E1 and [$^{18}F$]E2:

Column: Cosmosil (R) C18 column (from Nacalai Tesque, Inc)
Mobile phase: acetonitrile/water (4/6)
Flow rate: 1.0 mL/minute

The radiochemical purity and radiochemical yield of each of the synthesized $^{18}F$-labeled products were as follows:

[$^{18}F$]E3; radiochemical purity: 99% or more, radiochemical yield: 50%,
[$^{18}F$]D3; radiochemical purity: 98% or more, radiochemical yield: 10%,
[$^{18}F$]E1; radiochemical purity: 99% or more, radiochemical yield: 25%,
[$^{18}F$]E2; radiochemical purity: 99% or more, radiochemical yield: 25%.

Example 7: Autoradiography Using Postmortem Brain Section of Patient with Alzheimer's Disease

A postmortem autopsy brain section (temporal lobe) of a patient (92 years old, female) with Alzheimer's disease was incubated together with each of [$^{18}F$]E3, [$^{18}F$]D3, [$^{18}F$]E1, and [$^{18}F$]E2 (0.37 MBq/100 μL each) at room temperature for 1 hour. The resultant was washed with a 50 volume% ethanol aqueous solution (2 minutes × 2) and water (2 minutes × 2), and after drying, the section was contacted with BAS imaging plate (from Fujifilm Corporation) for 1.5 hours. The image of autoradiography was obtained using BAS5000 scanner system (from Fujifilm Corporation).

A section adjacent to the brain section used was immunostained in the following manner.
First, a paraffin-embedded brain section of the Alzheimer's disease patient (92 years old female, temporal lobe, 6 μm in thickness) was incubated with xylene (15 minutes × 2), ethanol (1 minute × 2), a 90 volume% ethanol aqueous solution (1 minute × 2), and a 70 volume% ethanol aqueous solution (1 minute × 1) in that order for complete deparaffinization, and then washed in water (2.5 minutes × 2). Thereafter, the section was subjected to autoclave treatment for 15 minutes in 0.01 mol/L citrate buffer solution (pH 6.0). The resultant was incubated in PBS-Tween20 (5 minutes × 2), and a mouse anti-$A\beta_{1-42}$ primary monoclonal antibody (from Wako Pure Chemical Industries Ltd.) was then added thereto, which was then incubated at room temperature overnight. The resultant was incubated in PBS-Tween20 (2 minutes × 3), and a biotin-goat anti-mouse IgG (from Funakoshi Co., Ltd.) was then added thereto, which was then incubated at room temperature for 1 hour. The resultant was incubated in PBS-Tween20 (5 minutes × 3), and a streptavidin-peroxidase complex (from Funakoshi Co., Ltd.) was added thereto, which was then incubated at room temperature for 30 minutes. In addition, the resultant was incubated in PBS-Tween20 (2 minutes × 3); 3,3'-diaminobenzidine was added thereto, which was then incubated at room temperature for 30 minutes; and after washing with water, the section was observed under a microscope to provide an immunostained image (FIG. 10).

Autoradiographic images of [$^{18}F$]E3, [$^{18}F$]D3, [$^{18}F$]E1, and [$^{18}F$]E2 are shown in FIGS. 6 to 9, respectively. FIG. 6 is the autoradiographic image of [$^{18}F$]E3; FIG. 7 is the autoradiographic image of [$^{18}F$]D3; FIG. 8 is the autoradiographic image of [$^{18}F$]E1; and FIG. 9 is the autoradiographic image of [$^{18}F$]E2. As shown in FIGS. 6 and 7, the gray matter was visualized in granules for [$^{18}F$]E3 and [$^{18}F$]D3. Amyloid plaques in Alzheimer's disease patients are generally known to be distributed in granules in the gray matter. The visualized sites for [$^{18}F$]E3 and [$^{18}F$]D3 were almost coincident with stained sites in the immunostaining image (FIG. 10). These results suggested that according to the experimental results, amyloid plaques were visualized for [$^{18}F$]E3 and [$^{18}F$]D3.

In contrast, as shown in FIGS. 8 and 9, [$^{18}F$] E1 and [$^{18}F$]E2 did not visualize the gray matter but rather strongly visualized the white matter, suggesting that they were not high in the ability to visualize amyloid.

Example 8: In Vivo Radioactivity Distribution Experiment

Through the tail vein of ddY mice (male, 5 week-old) was injected each of a 10 volume% ethanol-containing saline solution of [$^{18}F$]E3 (45.4 kBq, 100 μL), a 10 volume% ethanol-containing saline solution of [$^{18}F$]D3 (38.2 kBq,

100 μL), a 10 volume% ethanol-containing saline solution of [$^{18}$F]E1 (45.1 kBq, 100 μL), and a 10 volume% ethanol-containing saline solution of [$^{18}$F]E2 (40.6 kBq, 100 μL). Five mice were used for each group and sacrificed 2, 10, 30, and 60 minutes after administration to remove major organs. The blood and organs were weighed and further measured for radioactivity using a γ counter. The amount of radioactivity per unit mass was measured in each organ to evaluate radioactivity distribution.

The results are shown in Tables 3 to 6. As shown in these tables, each compound showed good transition into the brain early after administration and subsequent rapid radioactivity disappearance.

Table 3 Radioactivity Distribution in Each Organ for [$^{18}$F]E3 (SD in Parenthesis)

| Organ | Radioactivity Amount (% injected dose / g) | | | |
|---|---|---|---|---|
| | 2 min. after | 10 min. after | 30 min. after | 60 min. after |
| Blood | 2.26 (0.21) | 1.91 (0.68) | 2.42 (0.17) | 2.38 (0.25) |
| Liver | 11.9 (1.35) | 11.7 (0.80) | 7.11 (1.56) | 5.30 (0.82) |
| Kidney | 8.65 (0.88) | 5.19 (0.88) | 3.30 (0.27) | 3.51 (1.21) |
| Intestine | 3.24 (0.43) | 7.95 (1.28) | 16.2 (1.71) | 17.9 (3.64) |
| Spleen | 3.67 (0.40) | 2.89 (0.37) | 2.31 (0.21) | 1.74 (0.05) |
| Pancreas | 6.82 (0.80) | 3.51 (0.37) | 2.31 (0.19) | 1.60 (0.18) |
| Heart | 6.12 (0.79) | 3.48 (0.56) | 2.43 (0.12) | 2.22 (0.08) |
| Lung | 5.74 (0.48) | 4.02 (0.32) | 2.80 (0.15) | 2.20 (0.14) |
| Stomach* | 1.07 (0.20) | 2.54 (0.88) | 2.02 (0.63) | 1.42 (0.22) |
| Brain | 5.78 (0.60) | 3.82 (0.33) | 1.85 (0.09) | 1.48 (0.07) |
| Bone | 2.45 (0.43) | 2.20 (0.09) | 2.10 (0.39) | 2.02 (0.36) |
| *: % injected dose per organ | | | | |

Table 4 Radioactivity Distribution in Each Organ for [$^{18}$F]D3 (SD in Parenthesis)

| Organ | Radioactivity Amount (% injected dose / g) | | | |
|---|---|---|---|---|
| | 2 min. after | 10 min. after | 30 min. after | 60 min. after |
| Blood | 2.66 (0.50) | 2.29 (0.25) | 2.98 (0.29) | 3.26 (0.37) |
| Liver | 10.3 (1.00) | 9.83 (0.22) | 6.91 (0.81) | 6.65 (0.90) |
| Kidney | 10.7 (0.96) | 6.66 (0.45) | 4.19 (0.78) | 3.30 (0.39) |
| Intestine | 2.56 (0.10) | 5.27 (0.27) | 9.82 (1.43) | 12.3 (0.79) |
| Spleen | 2.86 (0.66) | 4.09 (0.33) | 2.66 (0.57) | 3.07 (0.68) |
| Pancreas | 6.55 (0.63) | 4.74 (0.42) | 2.73 (0.50) | 2.74 (0.19) |
| Heart | 8.50 (1.40) | 4.19 (0.47) | 3.29 (0.68) | 3.74 (0.72) |
| Lung | 8.94 (1.76) | 4.59 (1.07) | 3.51 (0.44) | 3.39 (0.14) |
| Stomach* | 1.09 (0.05) | 1.64 (0.28) | 1.98 (0.47) | 2.06 (0.46) |
| Brain | 5.67 (0.48) | 6.39 (0.51) | 3.73 (0.50) | 2.61 (0.37) |
| Bone | 2.65 (0.61) | 2.75 (0.36) | 2.82 (0.78) | 2.77 (1.27) |
| *: % injected dose per organ | | | | |

Table 5 Radioactivity Distribution in Each Organ for [$^{18}$F]E1

| Organ | Radioactivity Amount (% injected dose / g) | | | |
|---|---|---|---|---|
| | 2 min. after | 10 min. after | 30 min. after | 60 min. after |
| Blood | 2.71 (0.20) | 2.95 (0.14) | 3.44 (0.24) | 3.27 (0.22) |
| Liver | 9.18 (1.45) | 12.1 (1.82) | 8.76 (1.01) | 6.52 (0.93) |
| Kidney | 11.1 (1.59) | 6.36 (0.21) | 5.00 (0.52) | 3.30 (0.38) |
| Intestine | 4.22 (0.33) | 8.56 (1.29) | 13.4 (1.45) | 21.8 (5.00) |
| Spleen | 3.77 (0.52) | 4.86 (0.79) | 3.94 (0.45) | 3.71 (0.24) |
| Pancreas | 6.13 (0.57) | 4.98 (0.26) | 3.60 (0.57) | 2.73 (0.23) |
| Heart | 8.76 (1.24) | 5.30 (0.77) | 4.52 (0.38) | 4.53 (0.66) |
| Lung | 8.18 (1.21) | 5.67 (0.68) | 4.70 (0.41) | 4.05 (0.17) |
| Stomach* | 1.26 (0.05) | 1.98 (0.15) | 2.70 (0.53) | 2.97 (1.57) |
| Brain | 4.69 (0.46) | 3.12 (0.10) | 2.42 (0.22) | 1.99 (0.08) |
| Bone | 2.71 (0.53) | 2.65 (0.45) | 2.86 (0.41) | 2.84 (1.67) |
| *: % injected dose per organ | | | | |

Table 6 Radioactivity Distribution in Each Organ for [$^{18}$F]E2

| Organ | Radioactivity Amount (% injected dose / g) | | | |
|---|---|---|---|---|
| | 2 min. after | 10 min. after | 30 min. after | 60 min. after |
| Blood | 3.37 (0.46) | 3.83 (1.24) | 3.10 (0.34) | 3.40 (0.32) |
| Liver | 6.84 (1.50) | 11.7 (0.58) | 7.95 (1.32) | 6.95 (0.68) |
| Kidney | 11.8 (2.51) | 7.55 (0.36) | 3.92 (0.45) | 3.12 (0.41) |
| Intestine | 3.46 (0.75) | 12.5 (2.77) | 20.3 (2.55) | 22.1 (2.95) |
| Spleen | 2.71 (0.44) | 4.53 (0.62) | 3.33 (0.66) | 3.17 (0.62) |
| Pancreas | 5.42 (0.13) | 4.91 (0.95) | 2.60 (0.25) | 2.73 (0.31) |
| Heart | 7.42 (0.53) | 4.41 (0.17) | 8.53 (12.0) | 3.92 (0.64) |
| Lung | 6.71 (0.24) | 4.98 (0.56) | 3.34 (0.55) | 4.06 (0.99) |
| Stomach* | 1.13 (0.18) | 2.11 (0.55) | 1.56 (0.34) | 2.73 (2.44) |
| Brain | 5.96 (0.48) | 3.77 (0.40) | 2.18 (0.22) | 1.91 (0.15) |
| Bone | 1.86 (0.53) | 1.91 (0.54) | 2.07 (3.97) | 2.36 (1.16) |
| *: % injected dose per organ | | | | |

Example 9: Ex vivo Autoradiography Using Alzheimer's Disease Model Mouse

To confirm whether the compound 2 has the performance capable of visualizing amyloid plaques in the brain, an image was taken by ex vivo autoradiography in a Tg2576 mouse as one of Alzheimer's disease model animals.

[$^{18}$F]D3 (55.5 MBq (1.5 mCi)/200 $\mu$L) was administered to a Tg2576 mouse (24-month old, female) and a normal mouse (24-month old, female) through their tail vein. Each mouse was sacrificed 1 hour after administration, and the brain was removed, embedded in a carboxymethylcellulose solution, and frozen in a dry ice-hexane bath, followed by preparing frozen sections having a thickness of 30 $\mu$m using a cryostat (Leica). The resultant brain tissue sections were each placed on an imaging plate (Model BAS-SR, from Fujifilm Corporation) for 30 minutes for exposure, and the respective autoradiographs were obtained using an image analyzer (Bio Imaging Analyzer Model BAS5000, from Fujifilm Corporation). The same sections were each stained with thioflavin S (200 mol/L), washed, and observed

under a fluorescent microscope.

FIG. 11A shows ex vivo autoradiography in the brain of the Tg2576 mouse, and FIG. 12A shows ex vivo autoradiography in the brain of the normal mouse. As shown in these figures, many spots of radioactivity were detected on the ex vivo autoradiography of the brain in the Tg2576 mouse. These spots of radioactivity agreed with thioflavin S-stained images (FIG. 11B) in the same section. In contrast, no spots of radioactivity were detected which correspond to thioflavin S-stained images (FIG. 12B), in the ex vivo autoradiography of the normal mouse brain.
These results showed that [$^{18}$F]D3 could visualize amyloid plaques in the brain.

Names of compounds used in the following Examples and Comparative Examples were defined below as in Table 7.

Table 7 Name of Evaluation Compound Used in Example

| Compound Name | Common Name |
|---|---|
| [$^{18}$F]D1 | 4-(6-(2-[$^{18}$F]Fluoroethoxy)quinoxalin-2-yl)aniline |
| [$^{18}$F]D2 | 4-(6-(2-[$^{18}$F]Fluoroethoxy)quinoxalin-2-yl)-N-methylaniline |
| Compound 13 | 2-((2-(4-((Di-tert-Butoxycarbonyl)amino))phenyl)quinoxalin-6-yl)oxy)ethyl-4-methylbenzenesulfonate |
| Compound 14 | 2-((2-(4-((tert-Butoxycarbonyl)(methyl)amino))phenyl)quinoxalin-6-yl)oxy)ethyl-4-methylbenzenesulfonate |
| D1 | 4-(6-(2-Fluoroethoxy)quinoxalin-2-yl)aniline (Non-Radioactive Fluorine-Labeled Form of D1) |
| D2 | 4-(6-(2-Fluoroethoxy)quinoxalin-2-yl)-N-methylaniline (Non-Radioactive Fluorine-Labeled Form of D2) |

Reference Example 1: Synthesis of 4-(6-(2-Fluoroethoxy)quinoxalin-2-yl)aniline (D1)

The non-radioactive fluorine-labeled form of [$^{18}$F]D1 was synthesized as follows according to the scheme 8 shown in FIG. 13.

Step 9-1: Synthesis of 2-Chloroquinoxalin-6-ol (31)

6-Methoxy-2-chloroquinoxaline (97.3 mg, 0.5 mmol) was added to a toluene (4 mL) suspension of aluminum chloride (160.0 mg, 1.2 mmol) at 0°C, which was then stirred at 80°C for 4 hours. Then, the resultant was cooled down to 0°C, and the reaction was stopped using ice water. This was subjected to ethyl acetate extraction (60 mL × 2), and the organic layer was then dried with sodium sulfate. The solvent was evaporated and the residue was purified by silica-gel chromatography (chloroform/methanol = 30/1 (volume ratio)) to provide (31) shown in the scheme 8 (yield: 59.3 mg (65.7%)). $^1$H-NMR (400 MHz, deuterated methanol) δ 8.71 (s, 1H), 7.84 (d, J=9.2 Hz, 1H), 7.44 (dd, J=9.2, 2.8 Hz, 1H), 7.32 (d, J=2.8 Hz, 1H); MS (APCI) m/z 181[MH$^+$].

Step 9-2: Synthesis of 2-Chloro-6-(2-(fluoroethoxy)quinoxaline (32)

Potassium carbonate (297.2 mg, 2.15 mmol) was added to an N,N-dimethylformamide (18 mL) solution of 2-chloroquinoxalin-6-ol (194.2 mg, 1.08 mmol) synthesized in the step 9-1, which was then stirred at 80°C for 0.5 hour. Then, 2-fluoroethyl-4-methylbenzenesulfonate (460 μL) was added thereto, which was then stirred at 80°C for 1.5 hours. This suspension was cooled down to room temperature and extracted with ethyl acetate (60 mL × 2). The total organic layer was dried with magnesium sulfate. The solvent was evaporated and the residue was then purified by silica-gel chromatography (ethyl acetate/hexane = 1/3 (volume ratio) and further chloroform) to provide (32) shown in the scheme 8 (yield: 133.0 mg (54.3%)).
$^1$H-NMR (400 MHz, deuterated chloroform) δ 8.72 (s, 1H), 7.93 (d, J=9.2 Hz, 1H), 7.51 (dd, J=9.2, 2.8 Hz, 1H), 7.39 (d, J=2.8 Hz, 1H), 4.92-4.78 (m, 2H), 4.43-4.34 (m, 2H); MS (APCI) m/z 227[MH$^+$].

Step 9-3: Synthesis of 4-(6-(2-fluoroethoxy)quinoxalin-2-yl)aniline (E1)

To a toluene (30 mL)/ethanol (4.2 mL) solution of 2-chloro-6-(2-(fluoroethoxy)quinoxaline (829.8 mg, 3.66 mmol) synthesized in the step 9-2 were added 4-(4,4,5,5-tetramethyl-1,3,2-dioxaborolan-2-yl)aniline (882.9 mg, 4.03 mmol), tetrakis(triphenylphosphine)palladium (127.1 mg, 0.110 mmol), and a potassium carbonate aqueous solution (2.0 mol/L,

4.03 mL), which was then heated to reflux for 3 hours. The reaction solution was cooled down to room temperature and then extracted with ethyl acetate (150 mL × 2). The total organic layer was dried with magnesium sulfate. The solvent was evaporated and the residue was purified by silica-gel chromatography (chloroform/methanol = 30/1 (volume ratio) and further ethyl acetate/hexane = 1/1 (volume ratio)) to provide D1 (yield: 173.0 mg (16.7%)).
$^1$H-NMR (400 MHz, deuterated chloroform) δ 9.18 (s, 1H), 8.03-7.98 (m, 3H), 7.45 (dd, J=9.2, 2.8 Hz, 1H), 7.35 (d, J=2.8 Hz, 1H), 6.83 (d, J=8.4 Hz, 2H), 4.93-4.79 (m, 2H), 4.43-4.35 (m, 2H), 3.94 (s, 2H); HRMS (EI) m/z calcd for C16H14FN3O ($M^+$) 283.1121, found 283.1123.

Example 10: Synthesis of 4-(6-(2-fluoroethoxy)quinoxalin-2-yl)-N-methylaniline (D2)

The non-radioactive fluorine-labeled form of [$^{18}$F]D2 was synthesized as follows according to the scheme 9 shown in FIG. 13.

Step 10:

Sodium methoxide (5 mol/L methanol solution, 936 μL, 4.68 mmol) was slowly added at 0°C to a methanol (30 mL) suspension of D1 (330.8 mg, 1.17 mmol) synthesized in the step 9-3 and paraformaldehyde (189.5 mg, 6.31 mmol), which was then heated to reflux for 2 hours. Then, the resultant was cooled down to room temperature, and sodium boron hydride (221.3 mg, 5.85 mmol) was gently added thereto while stirring, which was again heated to reflux for 1.5 hours. After stopping the reaction using a saturated sodium hydrogencarbonate aqueous solution, methanol was evaporated under reduced pressure and the residue was extracted with ethyl acetate (70 mL × 2). The total organic layer was dried with magnesium sulfate, and the solvent was evaporated. The residue was purified by silica-gel column chromatography (ethyl acetate/hexane = 1/1 (volume ratio)) to provide D2 (yield: 188.1 mg (54.1%)).
$^1$H-NMR (400 MHz, deuterated chloroform) δ 9.19 (s, 1H), 8.05 (d, J=8.4 Hz, 2H), 7.99 (d, J=9.2 Hz, 1H), 7.44 (dd, J=9.2, 2.8 Hz, 1H), 7.35 (d, J=2.8 Hz, 1H), 6.74 (d, J=8.4 Hz, 2H), 4.92-4.79 (m, 2H), 4.43-4.34 (m, 2H), 4.05 (s, 1H), 2.93 (s, 3H); HRMS (EI) m/z calcd for C17H16FN3O (M+) 297.1277, found 297.1280.

Reference Example 2: Synthesis of 2-((2-(4-((di-tert-Butoxycarbonyl)amino)phenyl)quinoxalin-6-yl)oxy)ethyl-4-methylbenzenesulfonate (Compound). 4-(6-(2-Fluoroethoxy)quinoxalin-2-yl)-N-methylaniline (Compound 13)

The labeling precursor for D1 was synthesized as follows according to the scheme 10 shown in FIG. 14.

Step 11-1: Synthesis of 6-(2-((tertButyldimethylsilyl)oxy)ethoxy)-2-chloroquinoxaline (35)

Potassium carbonate (90.8 mg, 0.657 mmol) was added to an N,N-dimethylformamide (7 mL) solution of 2-chloroquinoxalin-6-ol (59.3 mg, 0.328 mmol) synthesized in the step 9-1, and the resultant suspension was stirred at 80°C for 0.5 hour. Then, (2-bromoethoxy)-tert-butyldimethylsilane (140 μL) was added thereto, which was then stirred at 80°C for 2.5 hours. The resultant was cooled down to room temperature and then extracted with ethyl acetate (60 mL × 2), and the total organic layer was dried with magnesium sulfate. The solvent was evaporated and the residue was purified by silica-gel column chromatography (ethyl acetate/hexane = 1/10 (volume ratio)) to provide (35) shown in the scheme 10 (yield: 101.5 mg (91.3%)).
$^1$H-NMR (400 MHz, deuterated chloroform) 8.71 (s, 1H), 7.90 (d, J=9.2 Hz, 1H), 7.47 (dd, J=9.2, 2.8 Hz, 1H), 7.39 (d, J=2.8 Hz, 1H), 4.21 (t, J=4.8 Hz, 2H), 4.06 (t, J=4.8 Hz, 2H), 0.92 (s, 9H), 0.12 (s, 6H); MS (APCI) m/z 339 [$MH^+$].

Step 11-2: Synthesis of 4-6-(2-((tertButyldimethylsilyl)oxy)ethoxy)quinoxalin-2-yl)aniline (36)

To a toluene (28 mL)/ethanol (3.8 mL) solution of 6-(2-((tert-butyldimethylsilyl)oxy)ethoxy)-2-chloroquinoxaline (1,113 mg, 3.28 mmol) synthesized in the step 11-1 were added 4-(4,4,5,5-tetramethyl-1,3,2-dioxaborolan-2-yl)aniline (719.5 mg, 3.28 mmol), tetrakis(triphenylphosphine)palladium (113.7 mg, 0.0984 mmol), and potassium carbonate (2.0 mol/L aqueous solution, 3.61 mL), which was then heated to reflux for 3 hours. Tetrakis(triphenylphosphine)palladium (113.7 mg, 0.0984 mmol) was added thereto, which was further heated to reflux for 1 hour. The resultant was cooled down to room temperature and then extracted with ethyl acetate (60 mL × 2), and the total organic layer was dried with magnesium sulfate. The solvent was evaporated and the residue was purified by silica-gel column chromatography (chloroform/methanol = 40/1 (volume ratio)) to provide (36) shown in the scheme 10 (yield: 1,425 mg (quantitative)).
$^1$H-NMR (400 MHz, deuterated chloroform) δ 9.17 (s, 1H), 8.02-7.96 (m, 3H), 7.41 (dd, J=9.2, 2.8 Hz, 1H), 7.36 (d, J=2.8 Hz, 1H), 6.83 (d, J=8.0 Hz, 2H), 4.21 (t, J=4.8 Hz, 2H), 4.07 (t, J=4.8 Hz, 2H), 3.95 (s, 2H), 0.93 (s, 9H), 0.13 (s, 6H) ).

Step 11-3: Synthesis of 6-(2-((tertButyldimethylsilyl)oxy)ethoxy)-2-(p-di(tert-butoxycarbonyl)aminophenyl)quinoxaline (37)

To tetrahydrofuran (18 mL) were added 4-6-(2-((tertbutyldimethylsilyl)oxy)ethoxy)quinoxalin-2-yl)aniline (698.0 mg, 1.765 mmol) synthesized in the step 11-2, di-tert-butyldicarbonate (3,852 mg, 17.65 mmol), N,N-dimethyl-4-aminopyridine (21.6 mg, 0.177 mmol), and triethylamine (295 $\mu$L), which was then heated to reflux for 3 hours, cooled down to room temperature, and extracted with ethyl acetate (70 mL $\times$ 2). The total organic layer was dried with magnesium sulfate. The solvent was evaporated and the residue was then purified by silica-gel column chromatography (ethyl acetate/hexane = 1/2 (volume ratio)) to provide (37) shown in the scheme 10 (yield: 528.7 mg (50.3%)).
$^1$H-NMR (400 MHz, deuterated chloroform) $\delta$ 9.25 (s, 1H), 8.18 (d, J=8.4 Hz, 2H), 8.03 (d, J=9.2 Hz, 1H), 7.46 (dd, J=9.2, 2.8 Hz, 1H), 7.40 (d, J=2.8, 1H), 7.32 (d, J=8.4 Hz, 2H), 4.23 (t, J=4.8 Hz, 2H), 4.08 (t, J=4.8 Hz, 2H), 1.44 (s, 18H), 0.93 (s, 9H), 0.13 (s, 6H).

Step 11-4: Synthesis of 6-(2-((tertButyldimethylsilyl)oxy)ethoxy)-2-(p-di(tert-butoxycarbonyl)aminophenyl)quinoxaline (Compound 13)

Tetrabutylammonium fluoride (1 mol/L tetrahydrofuran solution, 1.06 mL) was added at 0°C to a tetrahydrofuran (10 mL) solution of 6-(2-((tertbutyldimethylsilyl)oxy)ethoxy)-2-(p-di(tert-butoxycarbonyl)aminophenyl)quinoxaline (528.7 mg, 0.887 mmol) synthesized in the step 11-3, which was then heated up to room temperature and stirred for 2 hours. The resultant was extracted with ethyl acetate (50 mL $\times$ 2), and the organic layer was dried with magnesium sulfate. After transpirating the solvent, the residue was dissolved in pyridine (10 mL), to which were added para-toluenesulfonyl chloride (845.5 mg, 4.44 mmol) and 4-dimethylaminopyridine (10.8 mg, 0.0887 mmol), followed by stirring the resultant at room temperature for 5 hours. The solvent was evaporated under reduced pressure, and the residue was extracted with ethyl acetate (70 mL $\times$ 2). The total organic layer was dried with magnesium sulfate. The solvent was evaporated and the residue was purified by silica-gel column chromatography (ethyl acetate/hexane = 1/1 (volume ratio)) to provide compound 13 (yield: 338.2 mg (60.0%)).
$^1$H-NMR (400 MHz, deuterated chloroform) $\delta$ 9.24 (s, 1H), 8.18 (d, J=8.8 Hz, 2H), 8.01 (d, J=9.2 Hz, 1H), 7.83 (d, J=8.4 Hz, 2H), 7.34-7.28 (m, 6H), 4.49 (t, J=4.8 Hz, 2H), 4.34 (t, J=4.8 Hz, 2H), 2.42 (s, 3H), 1.44 (s, 18H); MS (APCI) m/z 636[MH$^+$].

Example 11: Synthesis of 2-((2-(4-((tert-Butoxycarbonyl)(methyl)amino))phenyl)quinoxalin-6-yl)oxy)ethyl-4-methylbenzenesulfonate (Compound 14)

The labeling precursor for D2 was synthesized as follows according to the scheme 11 shown in FIG. 15.

Step 12-1: Synthesis of 4-(6-(2-((tertButyldimethylsilyl)oxy)ethoxy)quinoxalin-2-yl)-N-methylaniline (39)

Sodium methoxide (5 mol/L methanol solution, 1.44 mL, 7.2 mmol) was slowly added at 0°C to a methanol (40 mL) suspension of 4-6-(2-((tertbutyldimethylsilyl)oxy)ethoxy)quinoxalin-2-yl)aniline (712.0 mg, 1.8 mmol) synthesized in the step 11-2 and paraformaldehyde (291.9 mg, 9.72 mmol), which was then heated to reflux for 2.5 hours. Then, the resultant was cooled down to room temperature, and sodium boron hydride (340.5 mg, 9.0 mmol) was gently added thereto, which was then heated to reflux for 1.5 hours. Thereafter, the reaction was stopped using a saturated sodium carbonate aqueous solution, and methanol was evaporated under reduced pressure. The residue was extracted with ethyl acetate (70 mL $\times$ 2); the total organic layer was dried with magnesium sulfate; and the solvent was evaporated. The residue was purified by silica-gel chromatography (ethyl acetate/hexane = 1/2 (volume ratio)) to provide (39) shown in the scheme 11 (yield: 118.8 mg (16.1%)). $^1$H-NMR (400 MHz, deuterated chloroform) $\delta$ 9.18 (s, 1H), 8.04 (d, J=8.4 Hz, 2H), 7.96 (d, J=9.2 Hz, 1H), 7.42-7.39 (m, 2H), 6.74 (d, J=8.4 Hz, 2H), 4.20 (t, J=4.8 Hz, 2H), 4.07 (t, J=4.8 Hz, 2H), 2.93 (s, 3H), 0.93 (s, 9H), 0.13 (s, 6H).

Step 12-2: tert-Butyl(4-(6-(2-((tertbutyldimethylsilyl)oxy)ethoxy)quinoxalin-2-yl)phenyl)(methyl)carbamate (40)

Di-tert-butyldicarbonate (632.9 mg, 2.90 mmol), 4-dimethylaminopyridine (3.54 mg, 0.0290 mmol), and triethylamine (48 $\mu$L) were added to a tetrahydrofuran (8 mL) solution of 4-(6-(2-((tertbutyldimethylsilyl)oxy)ethoxy)quinoxalin-2-yl)-N-methylaniline (118.8 mg, 0.290 mmol) synthesized in the step 13-1, which was then heated to reflux for 6 hours. The resultant was cooled down to room temperature and extracted with ethyl acetate (60 mL $\times$ 2); the total organic layer was dried with magnesium sulfate; and the solvent was evaporated. The residue was purified by silica-gel chromatography (ethyl acetate/hexane = 1/3 (volume ratio)) to provide (40) shown in the scheme 11 (yield: 111.2 mg (75.2%)).
$^1$H-NMR (400 MHz, deuterated chloroform) $\delta$ 9.23 (s, 1H), 8.13 (d, J=8.4 Hz, 2H), 8.02 (d, J=9.2 Hz, 1H), 7.47-7.39 (m,

4H), 4.23 (t, J=4.8 Hz, 2H), 4.08 (t, J=4.8 Hz, 2H), 3.33 (s, 3H), 1.49 (s, 9H), 0.93 (s, 9H), 0.13 (s, 6H); MS (APCI) m/z 510[$MH^+$].

Step 12-3: Synthesis of 2-((2-(4-((tert-Butoxycarbonyl)(methyl)amino)phenyl)quinoxalin-6-yl)oxy)ethyl-4-methylbenzenesulfonate (Compound 14)

Tetrabutylammonium fluoride (1 mol/L tetrahydrofuran solution, 262 μL) was added at 0°C to a tetrahydrofuran (5 mL) solution of tert-butyl(4-(6-(2-((tertbutyldimethylsilyl)oxy)ethoxy)quinoxalin-2-yl)phenyl)(methyl)carbamate (111.2 mg, 0.218 mmol) synthesized in the step 12-2, which was then returned to room temperature and stirred for 3 hours. The resultant was extracted with ethyl acetate (60 mL × 2); the organic layer was dried with sodium sulfate; and the solvent was evaporated. The residue was dissolved in pyridine (5 mL), and para-toluenesulfonyl chloride (207.8 mg, 1.09 mmol) and 4-dimethylaminopyridine (2.7 mg, 0.0218 mmol) were added thereto, which was then stirred at room temperature for 5 hours. The solvent was evaporated under reduced pressure; the residue was extracted with ethyl acetate (50 mL × 2); and the total organic layer was dried with magnesium sulfate. After evaporating the solvent, the residue was purified by silica-gel column chromatography (ethyl acetate/hexane = 1/1 (volume ratio)) to provide compound 14 (yield: 72.6 mg (60.6%)).
$^1$H-NMR (400 MHz, deuterated chloroform) δ 9.22 (s, 1H), 8.12 (d, J=8.4 Hz, 2H), 8.00 (d, J=9.2 Hz, 1H), 7.83 (d, J=8.4 Hz, 2H), 7.43 (d, J=8.4 Hz, 2H), 7.34-7.28 (m, 4H), 4.48 (t, J=4.8 Hz, 2H), 4.33 (t, J=4.8 Hz, 2H), 3.33 (s, 3H), 2.42 (s, 3H), 1.49 (s, 9H); MS (APCI) m/z 550[$MH^+$].

Example 12: Synthesis of $^{18}$F-Labeled Compound

[$^{18}$F] fluoride ion was produced via $^{18}$O(p, n) $^{18}$F reaction using a cyclotron (CYPRIS HM-12, from Sumitomo Heavy Industries, Ltd. (Tokyo)), and passed through Sep-Pak Light QMA cartridge (from Waters) as a $^{18}$O-concentrated water aqueous solution. This cartridge was dried with nitrogen gas, and [$^{18}$F]fluoride ion was eluted with a 66 mmol/L potassium carbonate solution (0.3 mL). Kryptofix 222 (10 mg) was added to a [$^{18}$F]fluoride ion aqueous solution, and the solvent was removed at 120°C in a stream of argon gas. The residue was azeotroped with 300 μL of dehydrated acetonitrile 3 times in a stream of nitrogen gas. An acetonitrile (200 μL) solution of the compound 13 or 14 (1.0 mg) was added to a reaction vessel containing the prepared [$^{18}$F]fluoride ion, heated at 100°C for 10 minutes, and then cooled. Then, a 1 mol/L hydrochloric acid aqueous solution (450 μL) was added thereto, which was then heated at 100°C for 10 minutes. Saturated sodium hydrogencarbonate (500 μL) was added thereto to adjust pH. The resultant was extracted with ethyl acetate, and the solvent was evaporated under reduced pressure. The residue was dissolved in acetonitrile (200 μL), which was passed through a filter. The resultant was purified using HPLC under the following conditions to obtain [$^{18}$F]D1 from the compound 13 and [$^{18}$F]D2 from the compound 14.
[$^{18}$F]D1 and [$^{18}$F]D2:

Column: Cosmosil (R) C18 column (from Nacalai Tesque, Inc.)
Mobile phase: acetonitrile/water (4/6)
Flow rate: 1.0 mL/minute

The synthesized [$^{18}$F]D1 had a radiochemical purity of 95% or more and a radiochemical yield of 38%, and the [$^{18}$F]D2 had a radiochemical purity of 95% or more and a radiochemical yield of 18%. [$^{18}$F]D1 and [$^{18}$F]D2 were identified by confirming that they had the same HPLC retention times as those of the respective non-radioactive compounds (D1 and D2).

Example 13: Binding Evaluation Using Aβ Peptide Aggregation in Solution

$A\beta_{1-42}$ peptide (Peptide Institute, Inc., Osaka) was gently dissolved at a concentration of 0.25 mg/mL in a PBS solution (pH 7.4), which was then incubated at 37°C for 42 hours to prepare an $A\beta_{1-42}$ aggregate PBS solution.
Mixed were 50 μL of an ethanol solution of D1 or D2 (final concentration: 0.5 pmol/L to 25 μmol/L), 50 μL of an ethanol solution of [$^{125}$I]IMPY prepared by the method described in Example 5 (final concentration: 0.025 nmol/L, 2.0 kBq), 50 μL of a PBS solution of the $A\beta_{1-42}$ aggregates (final concentration: 0.125 μg/mL), and 850 μL of a 10 volume% ethanol aqueous solution, which was then incubated at room temperature for 3 hours. Thereafter, the resultant was filtered through Whatman (R) GF/B filter using a Brandell M-24 cell harvester, and the radioactivity of the filter was measured using a γ counter (Wallac 1470 Wizard, from Perkin-Elmer).
The half inhibition concentration ($IC_{50}$) value was determined from a replacement straight line using GraphPad Prism 5.0, and the inhibition constant (Ki) was calculated using the Cheng-Prusoff equation: the above-described Expression (1).

The Ki value of each compound is shown in Table 8. The binding affinity of D2 was 10 times or more higher than that of D1.

Table 8 Ki Value of Each Compound (Each value is in nmol/L and the value following ± shows SE)

| Compound | $K_I$ (nM) |
|---|---|
| D1 | 242 ± 29.0 |
| D2 | 15.7 ± 1.28 |

Example 14: In vitro Autoradiography Using Postmortem Brain Section from Alzheimer's Disease

A postmortem autopsy brain tissue of a patient (78 years old, female) with Alzheimer's disease identified by postmortem dissection was obtained from Graduate School of Medicine, Kyoto University. The presence and location of senile plaques in the section were confirmed by immunohistochemical staining using an anti-$A\beta_{1\text{-}42}$ antibody. The section was incubated together with $[^{18}F]$D1 or $[^{18}F]$D2 (0.37 MBq/100 μL) at room temperature for 1 hour. The resultant was immersed in a 50 volume% ethanol aqueous solution (2 minutes × 2) and washed with water (2 minutes × 2). After drying, the section was exposed to BAS imaging plate (from Fujifilm Corporation) for 1.5 hours. The image of autoradiography was obtained using BAS5000 scanner system (from Fujifilm Corporation).

The results are shown in FIGS. 16 and 17. FIG. 16 is an autoradiographic image of $[^{18}F]$D1. FIG. 17 is an autoradiographic image of $[^{18}F]$D2. $[^{18}F]$D2 provided a strong signal in a cerebral cortex area of the Alzheimer's brain section, provided a low degree of background in the white matter, and provided a strong marker for Aβ plaques. Conversely, $[^{18}F]$D1 showed high non-specific binding to the white matter in the Alzheimer's disease brain and was not observed to significantly bind to Aβ plaques. These autoradiographic results were intimately associated with the results of the in vitro binding evaluation.

Example 15: Immunohistochemical Staining of Senile Plaque in Human Alzheimer's Disease Brain Section

A postmortem autopsy brain tissue of a patient (78 years old, female) with Alzheimer's disease identified by postmortem dissection was obtained from Graduate School of Medicine, Kyoto University, and serial sections 6 μm thick of a paraffin-embedded brain tissue were used. First, they were each incubated with xylene (15 minutes × 2), ethanol (1 minute × 2), a 90 volume% ethanol aqueous solution (1 minute × 2), and a 70 volume% ethanol aqueous solution (1 minute × 1) in that order to perfectly perform deparaffinization, and then washed in water (2.5 minutes × 2). Thereafter, for antibody recovery, the section was subjected to autoclave treatment for 15 minutes in 0.01 mol/L citrate buffer solution (pH 6.0). The resultant was incubated in PBS-Tween20 (5 minutes × 2), and, for antibody recovery, incubated in a 90 volume% formic acid aqueous solution at room temperature for 5 minutes. The section was washed for 5 minutes with flowing tap water, and then incubated in PBS-Tween20 (2 minutes × 1). The section was incubated with a mouse anti-$A\#_{1\text{-}42}$ monoclonal primary antibody (from Wako Pure Chemical Industries Ltd.) at room temperature overnight. The resultant was incubated in PBS-Tween20 (5 minutes × 3) and then incubated together with biotin goat antimouse IgG (from Funakoshi Co., Ltd.) at room temperature for 1 hour. This was incubated in PBS-Tween20 (5 minutes × 2) and TBS (5 minutes × 1) and then incubated together with diaminobenzidine (DAB) as a chromogen for 5 minutes. After washing with water, the section was observed under a microscope (BIOREVO BZ-9000, from Keyence Corporation). The results are shown in FIG. 18B. FIG. 18A shows a close up of FIG. 17. The cumulative radioactivity of $[^{18}F]$D2 corresponded to the results of antibody immunohistochemical staining using the anti-$A\beta_{1\text{-}42}$ antibody.

Example 16: In vivo Radioactivity Distribution Experiment in Normal Mouse

An ethanol (10 μL)-containing saline solution of $[^{18}F]$D1 (25.8 kBq) or $[^{18}F]$D2 (48.2 kBq) (100 μL) was injected into the tail vein of ddY mice (male, 5-week old). Five mice were used for each group and sacrificed 2, 10, 30, and 60 minutes after administration to remove major organs. The blood and organs were weighed and further measured for radioactivity using a γ counter (Wallac 1470 Wizard 3, from Perkin-Elmer). The percent injected dose per organ was calculated by comparing tissue counts with suitably diluted aliquots of the injected material. The percent injected dose/g of each sample was calculated by comparing sample counts with the counts of the diluted initial dose. The results are shown in Tables 9 and 10.

Table 9 [$^{18}$F]D1 Each value shows the average value for 5 mice (SD).

| Organ | Radioactivity Amount (% injected dose / g) | | | |
|---|---|---|---|---|
| | 2 min. after | 10 min. after | 30 min. after | 60 min. after |
| Blood | 3.86 (0.48) | 4.39 (0.49) | 4.66 (0.13) | 5.10 (0.36) |
| Liver | 5.30 (1.33) | 6.58 (0.47) | 4.90 (0.29) | 4.88 (0.30) |
| Kidney | 11.92 (2.02) | 7.08 (0.69) | 4.67 (0.50) | 3.93 (0.39) |
| Intestine | 3.69 (0.44) | 5.48 (0.85) | 6.67 (0.52) | 8.19 (0.69) |
| Spleen | 3.54 (1.53) | 5.36 (1.35) | 4.55 (0.31) | 4.81 (0.75) |
| Pancreas | 6.21 (0.99) | 5.08 (0.78) | 3.76 (0.37) | 3.94 (0.47) |
| Heart | 7.55 (0.95) | 5.35 (0.70) | 5.07 (0.39) | 6.10 (0.35) |
| Lung | 8.28 (0.96) | 5.60 (0.67) | 5.21 (0.17) | 5.56 (0.43) |
| Stomach* | 1.68 (0.26) | 2.67 (0.32) | 3.31 (0.59) | 2.71 (0.30) |
| Brain | 7.59 (0.82) | 5.63 (0.25) | 3.50 (0.24) | 3.08 (0.12) |
| Bone | 2.01 (0.43) | 2.57 (0.50) | 3.23 (0.56) | 5.40 (1.06) |
| * % injected dose per organ | | | | |

Table 10 [$^{18}$F]D2 Each value shows the average value for 5 mice (SD).

| Organ | Radioactivity Amount (% injected dose / g) | | | |
|---|---|---|---|---|
| | 2 min. after | 10 min. after | 30 min. after | 60 min. after |
| Blood | 3.91 (0.22) | 3.35 (0.43) | 3.83 (0.18) | 4.15 (0.31) |
| Liver | 8.81 (1.00) | 9.29 (0.40) | 7.07 (0.19) | 6.10 (0.41) |
| Kidney | 9.86 (0.77) | 6.40 (0.62) | 4.14 (0.31) | 3.08 (0.21) |
| Intestine | 3.40 (0.34) | 8.06 (0.95) | 12.13 (2.05) | 13.37 (1.03) |
| Spleen | 3.14 (0.55) | 3.76 (0.55) | 3.07 (0.32) | 2.99 (0.21) |
| Pancreas | 6.47 (0.75) | 3.88 (0.52) | 2.72 (0.29) | 2.64 (0.34) |
| Heart | 6.12 (0.45) | 3.93 (0.40) | 3.20 (0.32) | 3.80 (0.22) |
| Lung | 8.13 (1.13) | 5.10(0.74) | 3.94 (0.33) | 3.92 (0.11) |
| Stomach* | 1.32 (0.21) | 1.86 (0.38) | 2.44 (0.62) | 2.00 (0.76) |
| Brain | 6.19 (0.30) | 5.11 (0.28) | 2.73 (0.16) | 2.41 (0.12) |
| Bone | 2.81 (0.52) | 2.12 (0.11) | 2.86 (0.45) | 4.26 (0.29) |
| * % injected dose per organ | | | | |

[$^{18}$F]D1 and [$^{18}$F]D2 were each observed to have a high brain uptake of 6.19 to 7.59% ID/g 2 minutes after administration, and thereafter the amount of radioactivity in the brain decreased (to 2.41 to 3.08% ID/g 60 minutes after administration). Because the brain of normal mice has no Aβ plaques, the initial high uptake and subsequent rapid disappearance thereof in the normal mouse brain are very promising characteristics for the in vivo image of Aβ plaques.

The compounds according to the above embodiment can be used in the technical field of diagnostic agents, particularly a diagnostic imaging agent.

It is apparent that the present invention is not limited to the above embodiment and the above example, and may be modified and changed without departing from the scope and spirit of the invention.

Provided is a compound effective as a diagnostic imaging probe targeting amyloid and an agent for Alzheimer's disease diagnosis including the compound.

**Claims**

1. A compound represented by the following general formula (1) or a salt thereof:

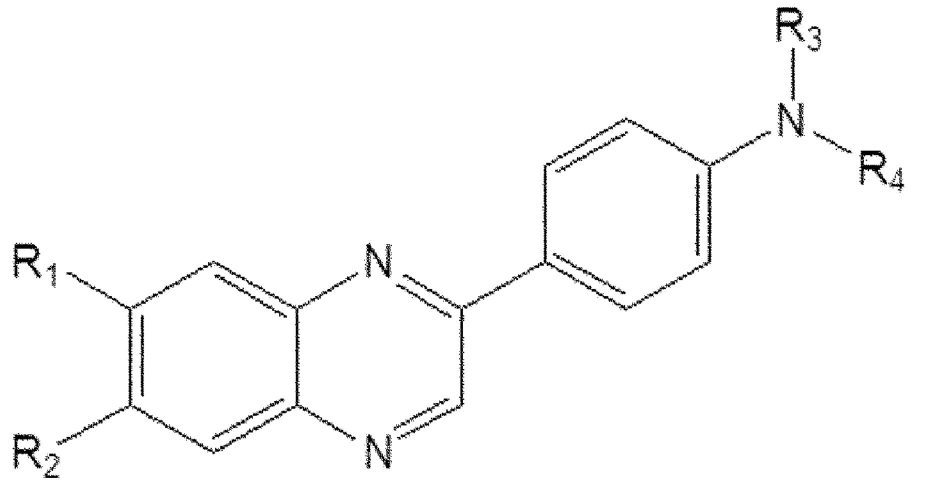

(1)

wherein one of $R_1$ and $R_2$ represents a 2-[$^{18}$F]fluoroethoxy group and the other represents a hydrogen atom; $R_3$ and $R_4$ each independently represent a hydrogen atom or a methyl group with the proviso that the cases where $R_3$ and $R_4$ each represent a hydrogen atom; and where $R_1$ represents a 2-[$^{18}$F]fluoroethoxy group, $R_2$ and $R_3$ each represent a hydrogen atom, and $R_4$ represents a methyl group are excluded.

2. The compound according to claim 1, wherein the compound is represented by the following general formula (2) or a salt thereof:

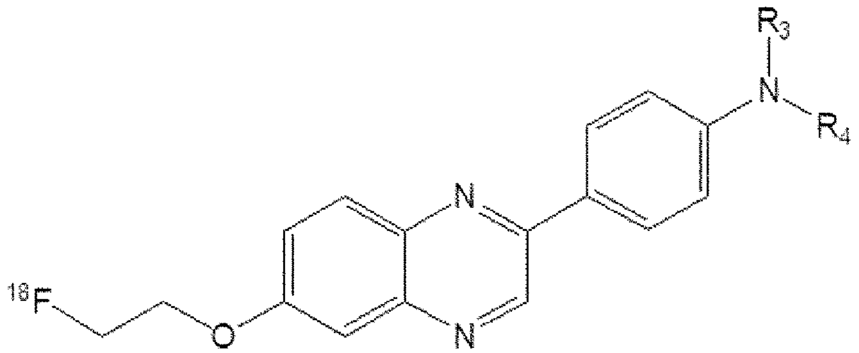

(2)

wherein $R_3$ and $R_4$ each independently represent a hydrogen atom or a methyl group with the proviso that the case where $R_3$ and $R_4$ each represent a hydrogen atom is excluded.

3. The compound according to claim 1, wherein the compound represented by the following formula (3):

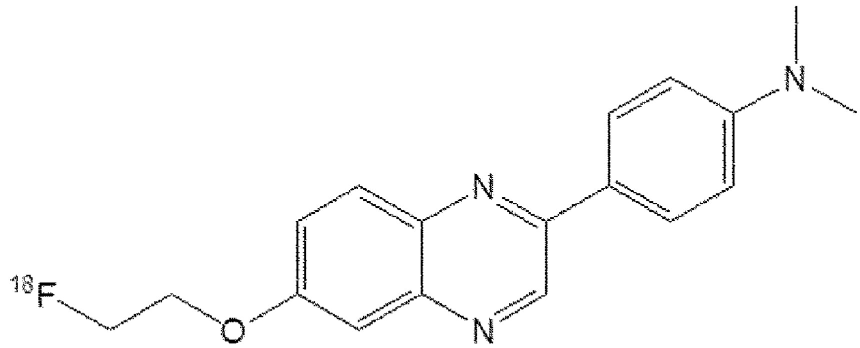

(3)

or (5) :

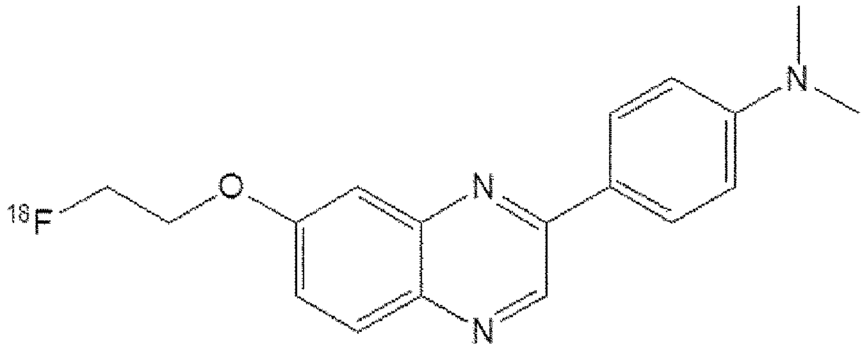

(5)

or a salt thereof.

**4.** A compound represented by the following general formula (6) or a salt thereof:

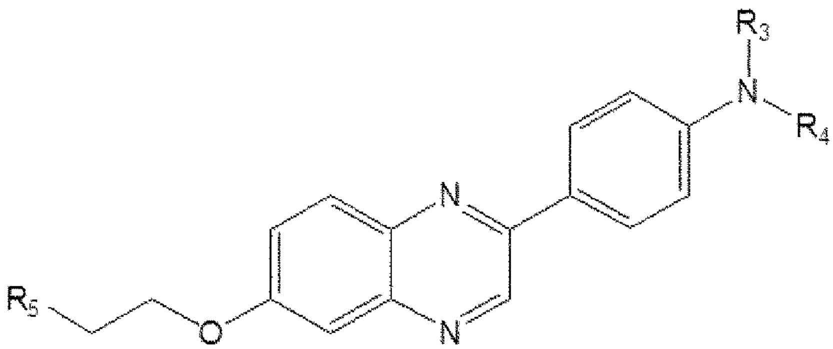

(6)

wherein $R_3$ and $R_4$ each independently represent a hydrogen atom or a methyl group (with the proviso that the case where $R_3$ and $R_4$ each represent a hydrogen atom is excluded), and $R_5$ represents a methanesulfonyloxy group, a trifluoromethanesulfonyloxy group, or an aromatic sulfonyloxy group.

**5.** The compound according to claim 4, wherein the compound represented by the following formula (7):

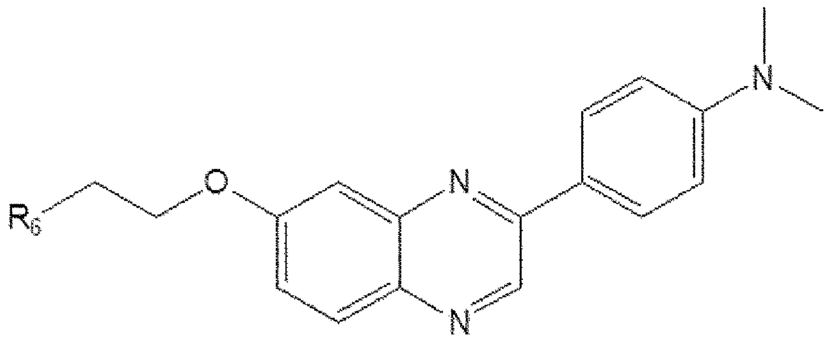

(7)

wherein $R_6$ represents a methanesulfonyloxy group, a trifluoromethanesulfonyloxy group, or an aromatic sulfonyloxy group
or (9):

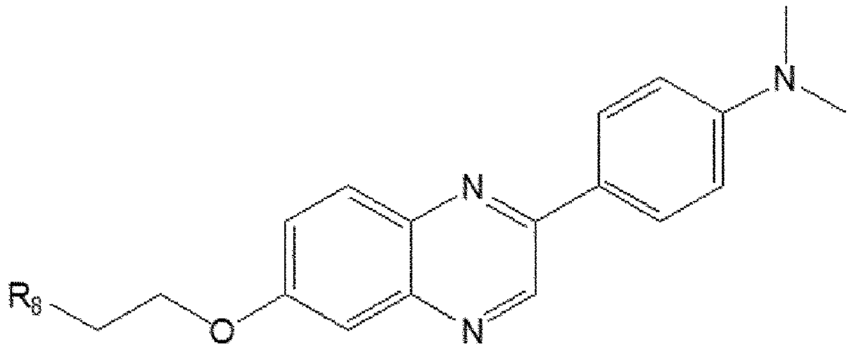

(9)

wherein $R_8$ represents a methanesulfonyloxy group, a trifluoromethanesulfonyloxy group, or an aromatic sulfonyloxy group
or a salt thereof.

**6.** An agent for amyloidosis diagnosis comprising a compound represented by the following general formula (1):

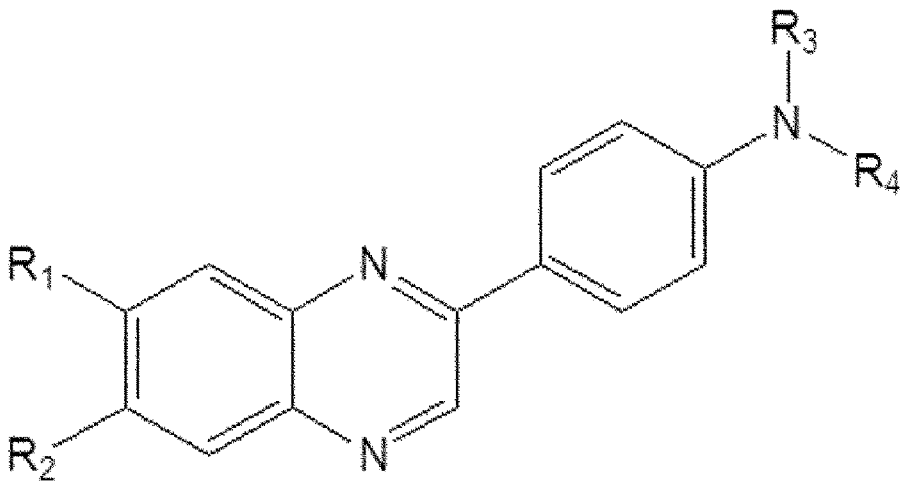

(1)

wherein one of $R_1$ and $R_2$ represents a 2-[$^{18}$F]fluoroethoxy group and the other represents a hydrogen atom; $R_3$ and $R_4$ each independently represent a hydrogen atom or a methyl group with the proviso that the cases where $R_3$ and $R_4$ each represent a hydrogen atom; and where $R_1$ represents a 2-[$^{18}$F]fluoroethoxy group, $R_2$ and $R_3$ each represent a hydrogen atom, and $R_4$ represents a methyl group are excluded.

**7.** The agent for amyloidosis diagnosis according to claim 6, wherein the compound represented by the following formula (3):

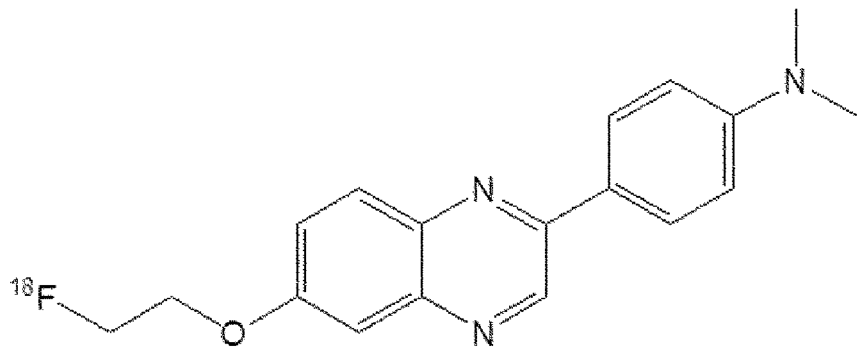

(3)

or (5) :

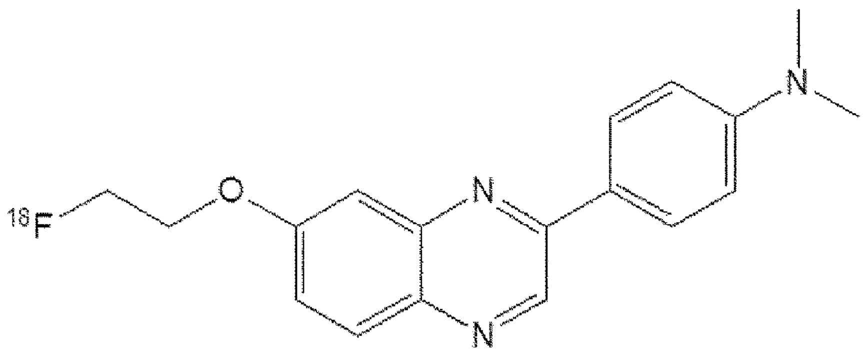

(5)

.

FIG. 1

SCHEME 1

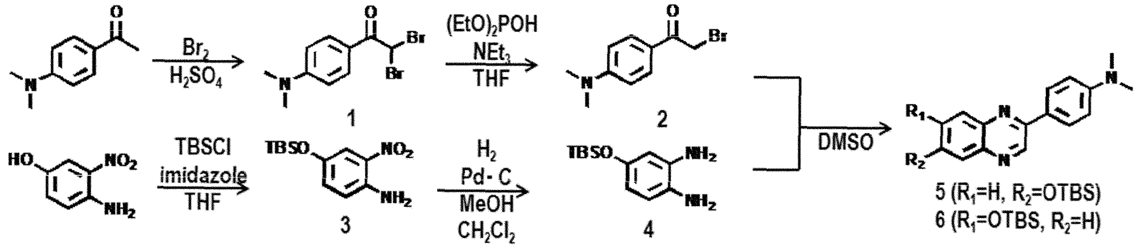


1 2 3 4

5 ($R_1$=H, $R_2$=OTBS)
6 ($R_1$=OTBS, $R_2$=H)

SCHEME 2

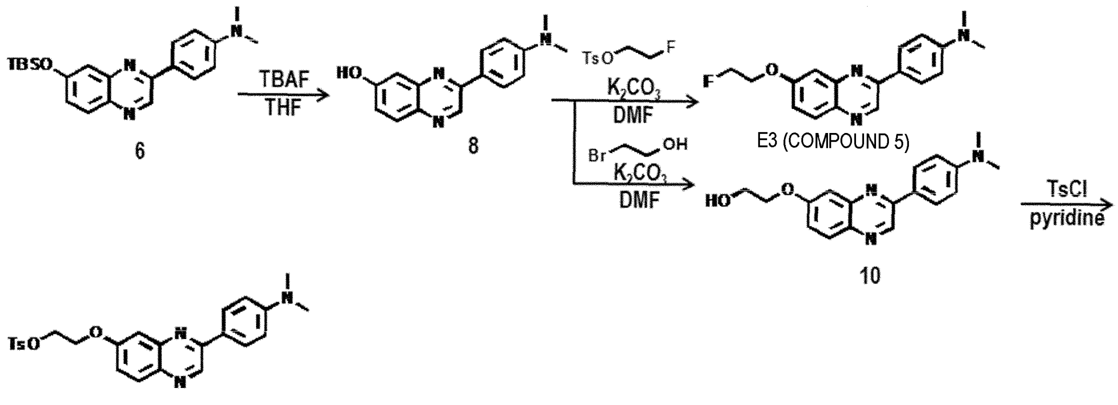


6 8 E3 (COMPOUND 5) 10

COMPOUND 3

FIG. 2

SCHEME 3

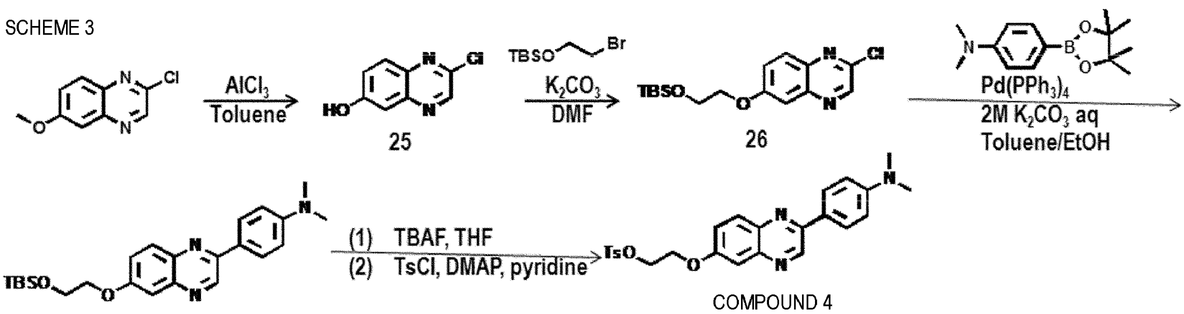


25

26

27

COMPOUND 4

SCHEME 4

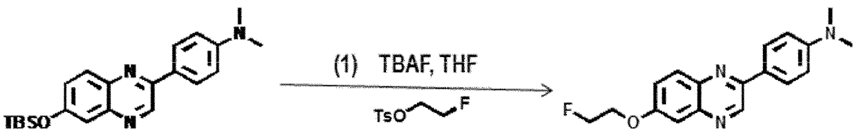


5

D3 (COMPOUND 6)

FIG. 3

SCHEME 5

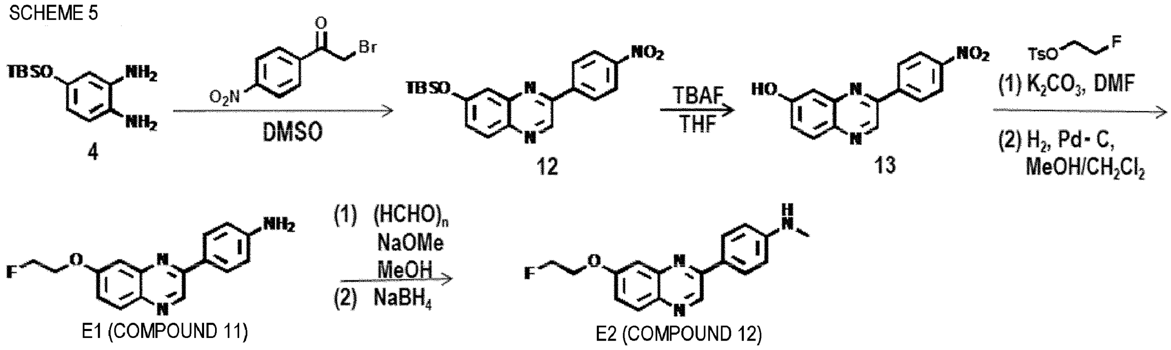


E1 (COMPOUND 11)

E2 (COMPOUND 12)

FIG. 4

SCHEME 6

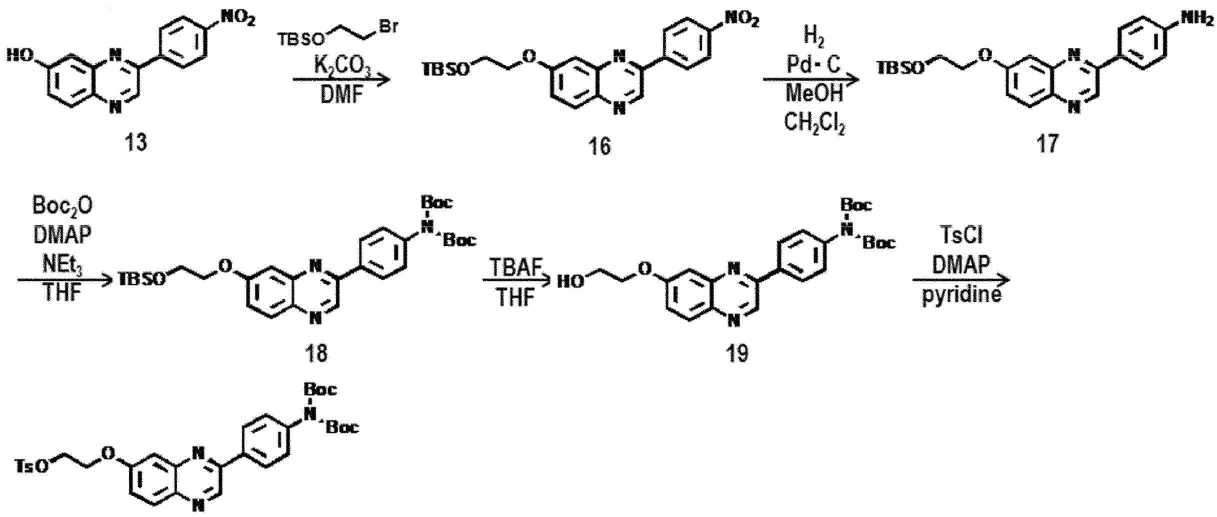


13

16

17

18

19

COMPOUND 9

FIG. 5

SCHEME 7

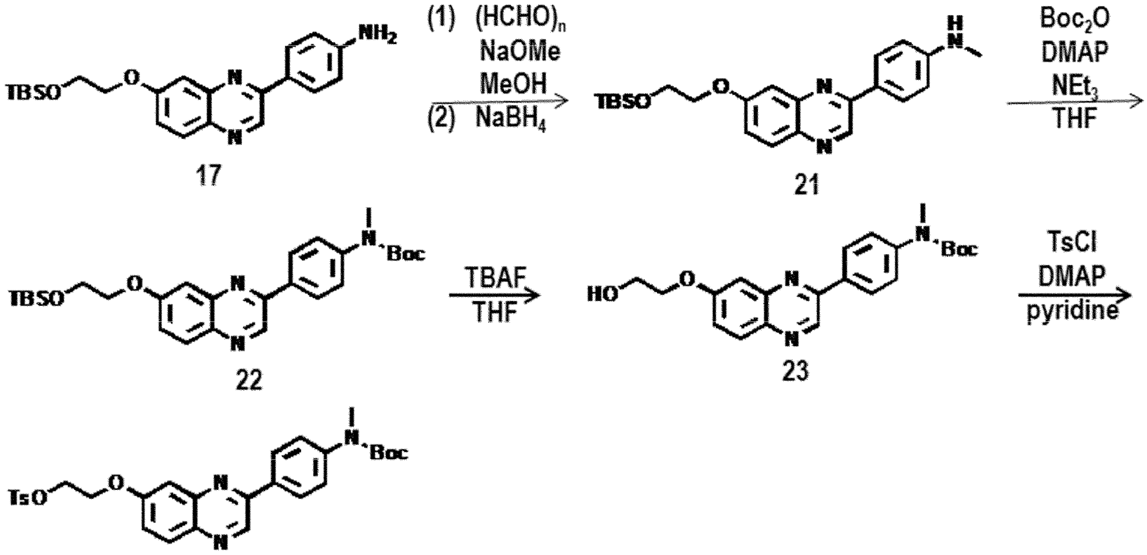


17

21

22

23

COMPOUND 10

FIG. 6

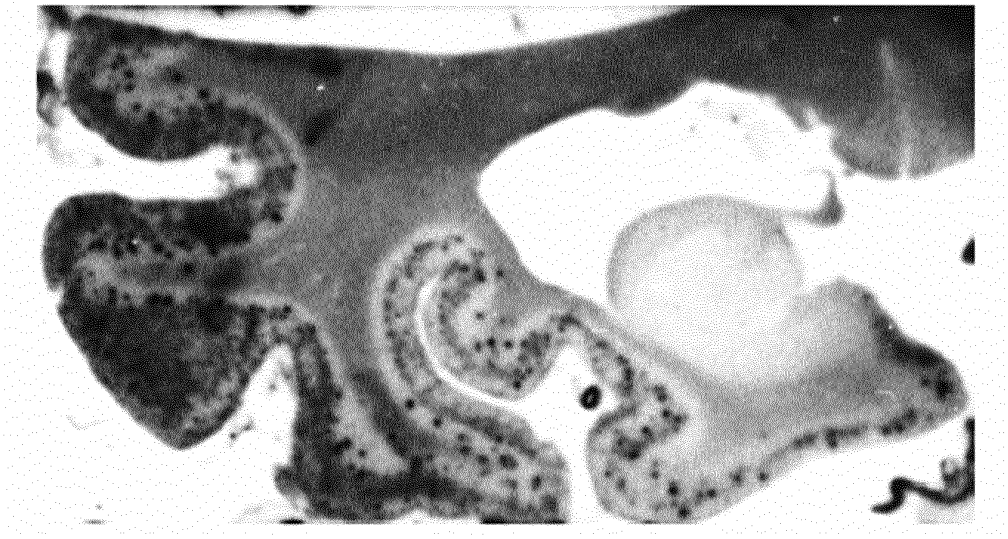

FIG. 7

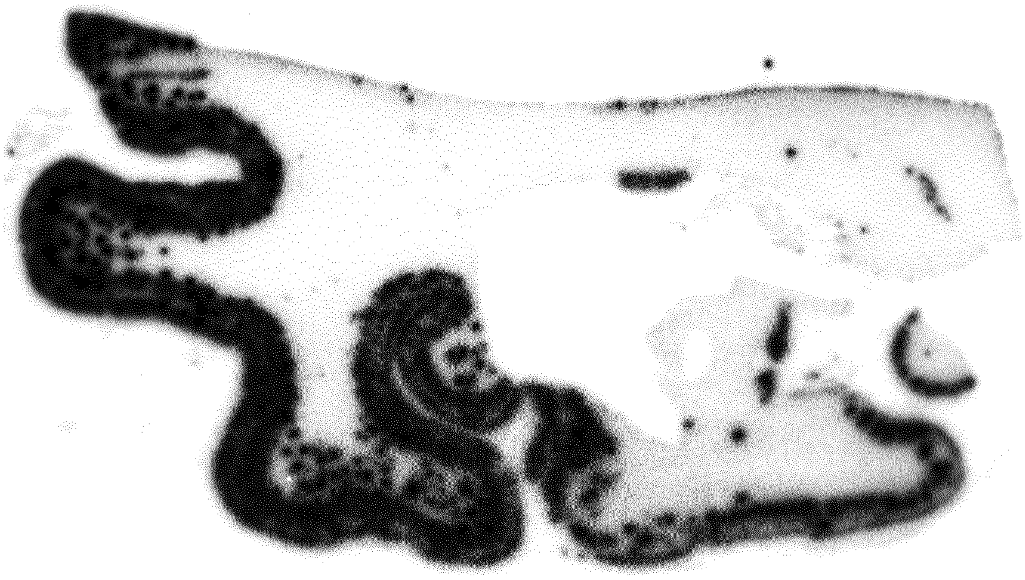

FIG. 8

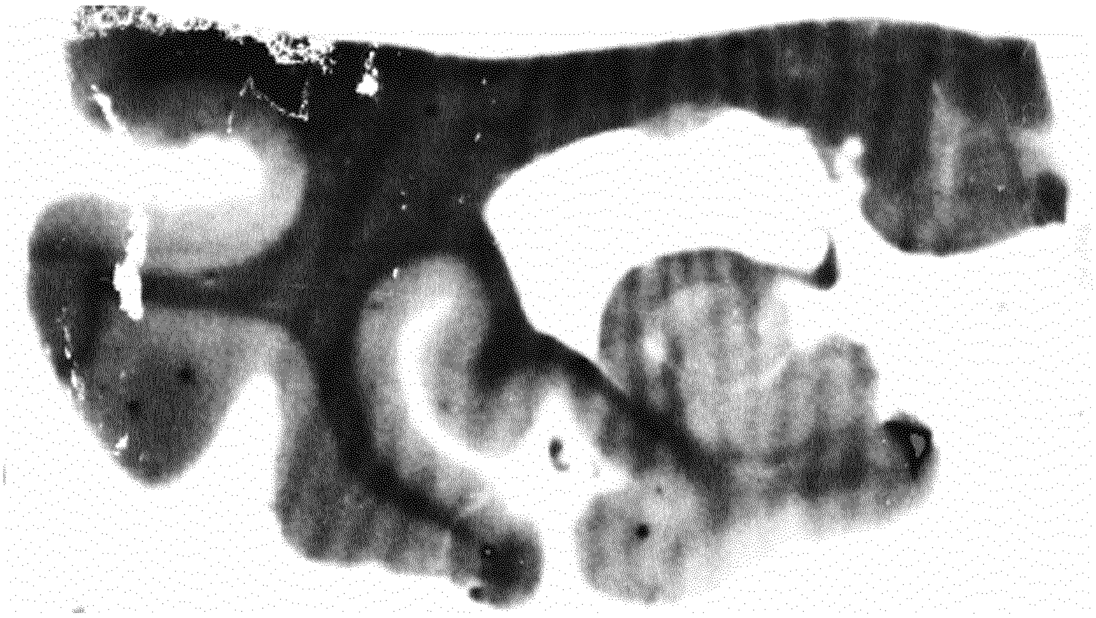

FIG. 9

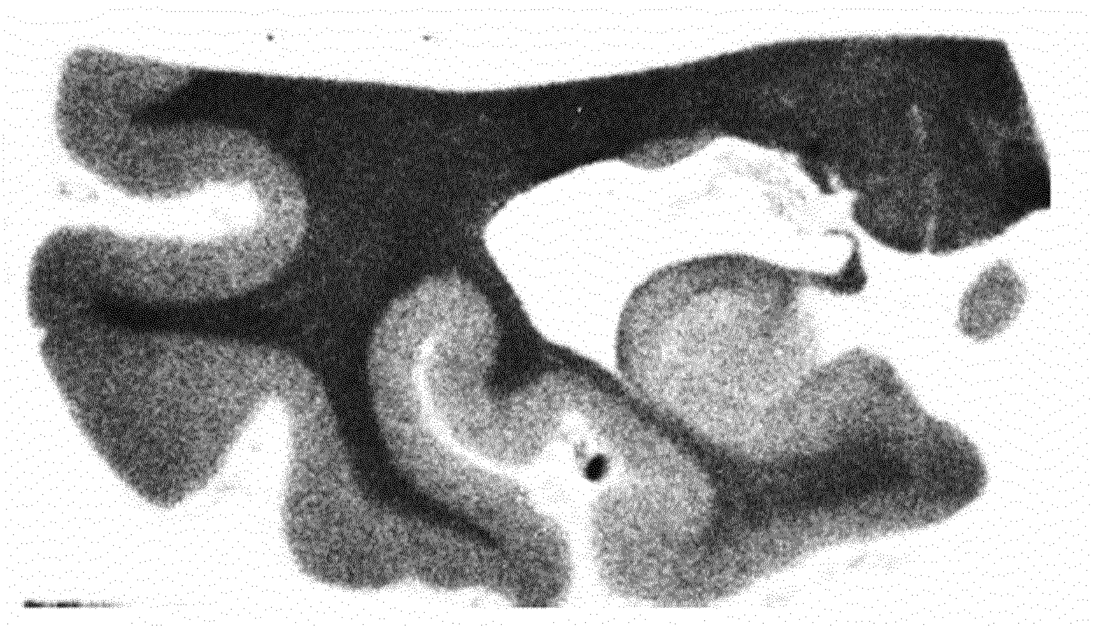

FIG. 10

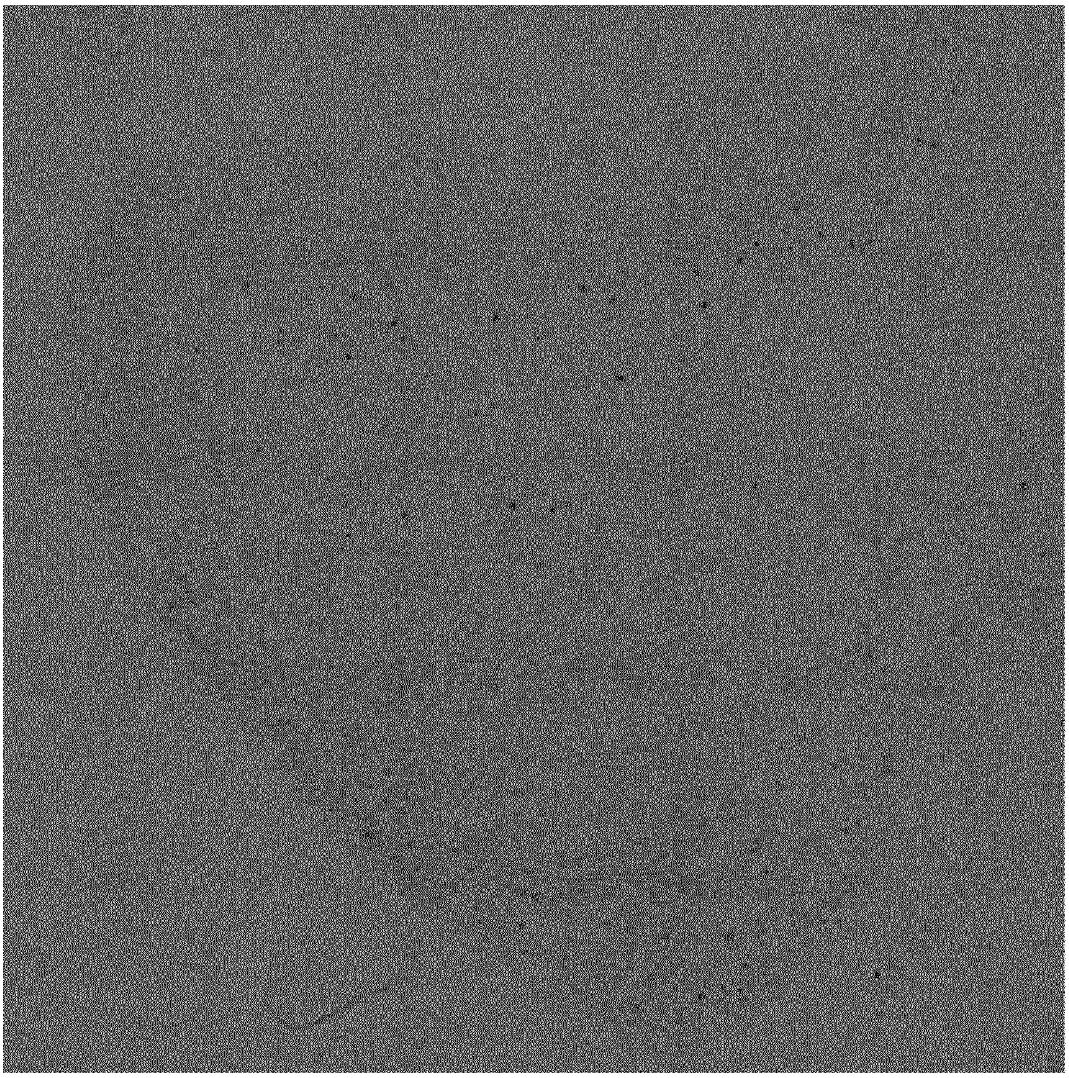

FIG. 11A

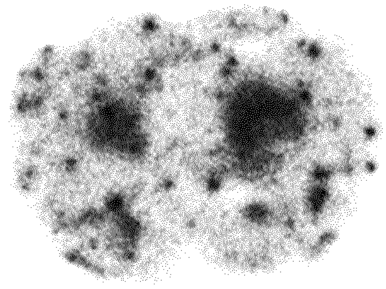

FIG. 11B

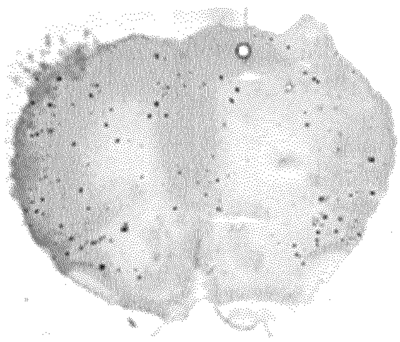

FIG. 12A

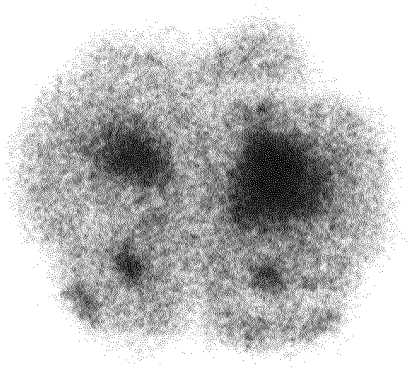

FIG. 12B

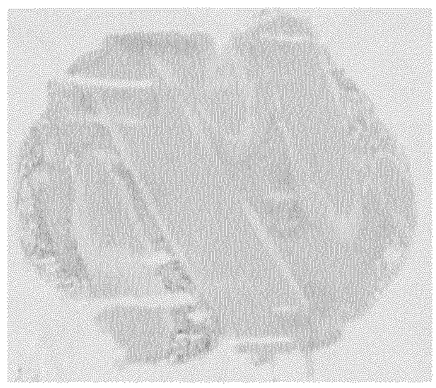

FIG. 13

SCHEME 8

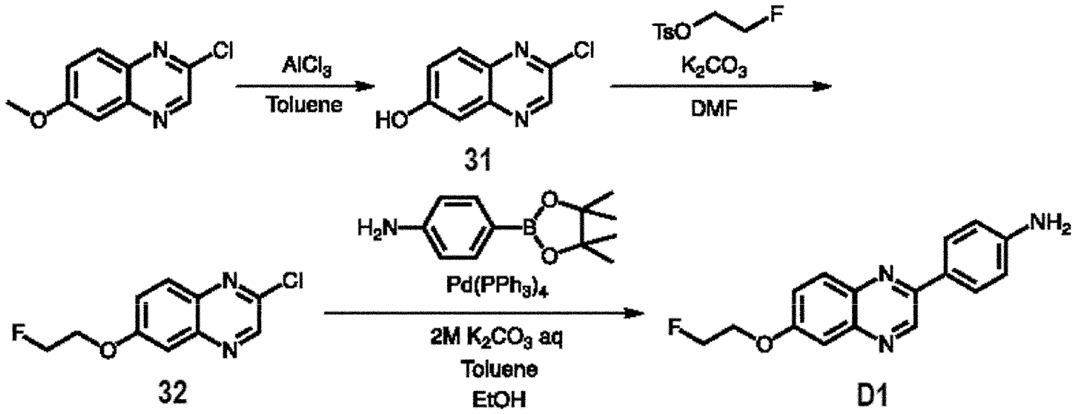


SCHEME 9

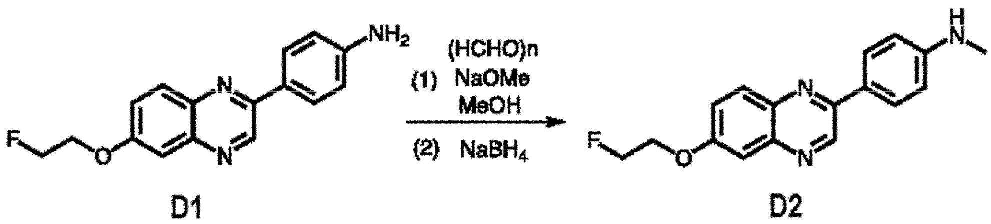

FIG. 14

SCHEME 10

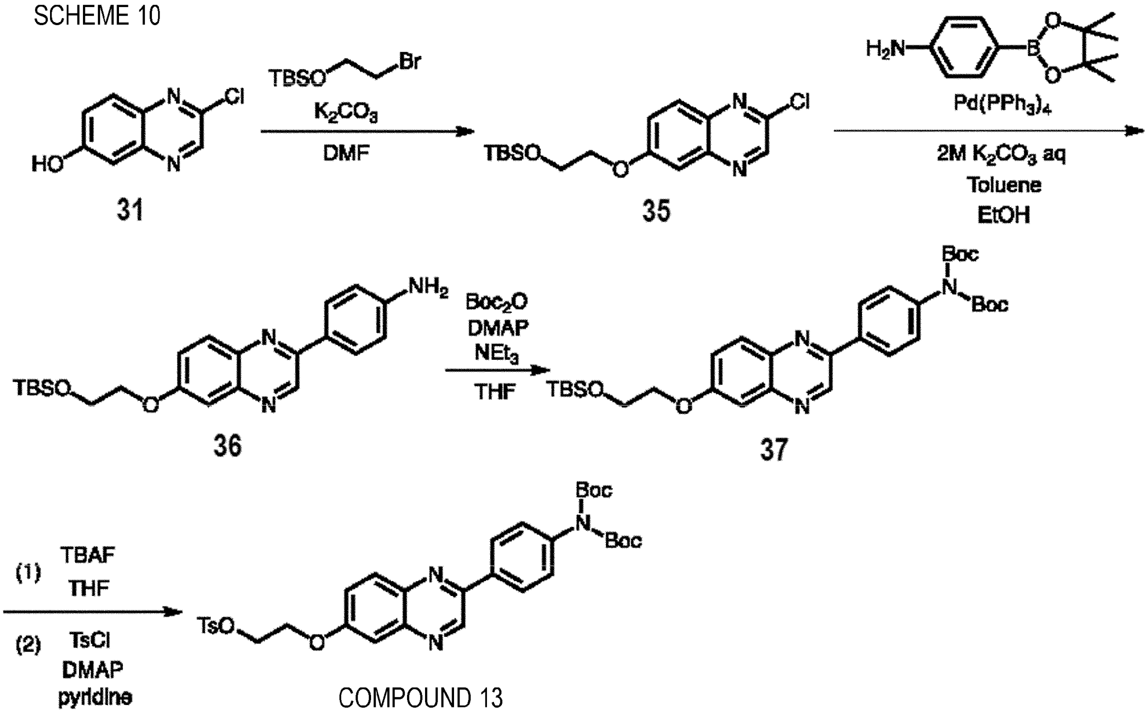


31

35

36

37

COMPOUND 13

FIG. 15

SCHEME 11

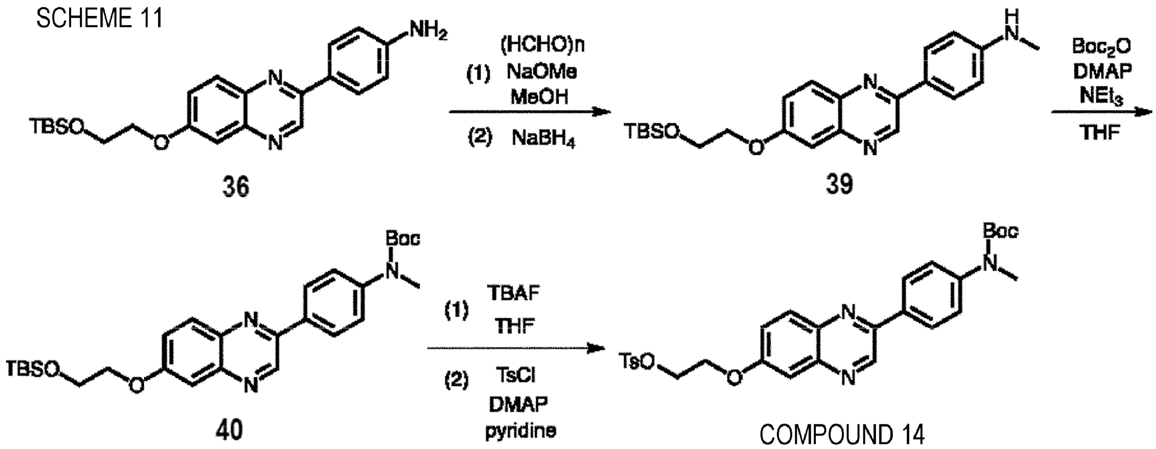


36

39

40

COMPOUND 14

FIG. 16

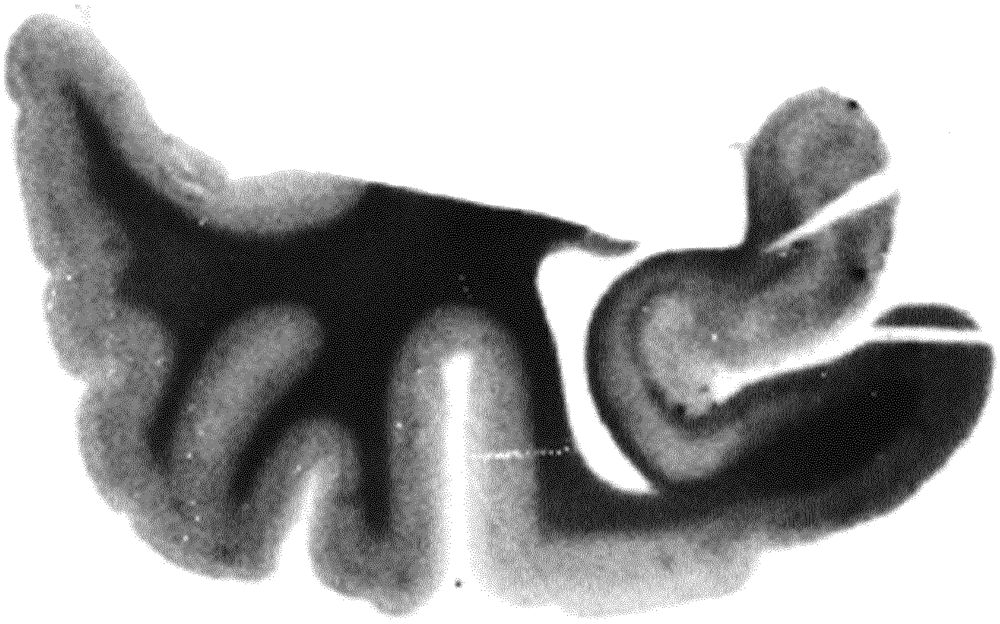

FIG. 17

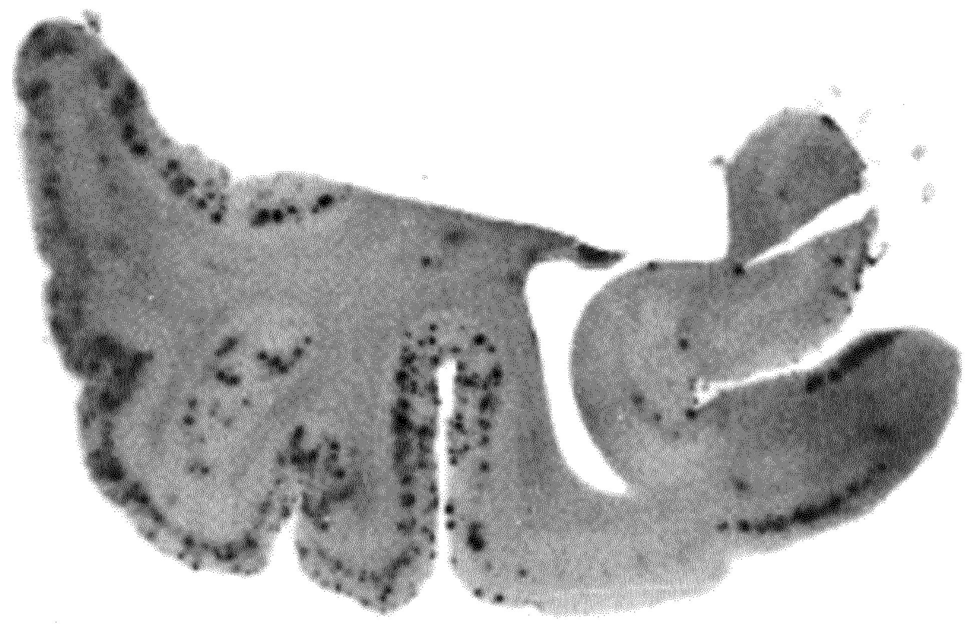

FIG. 18A

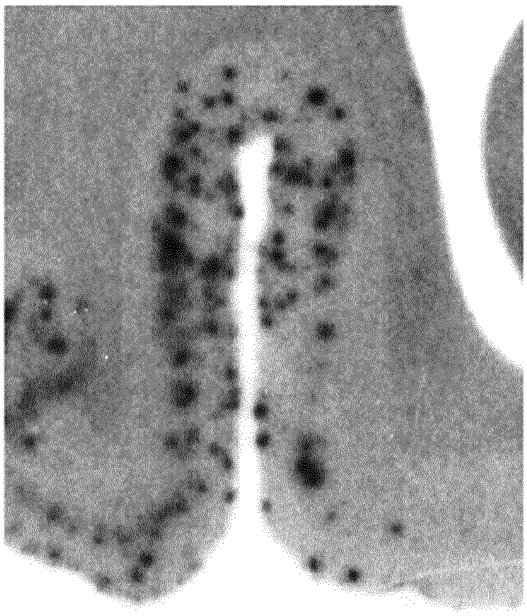

FIG. 18B

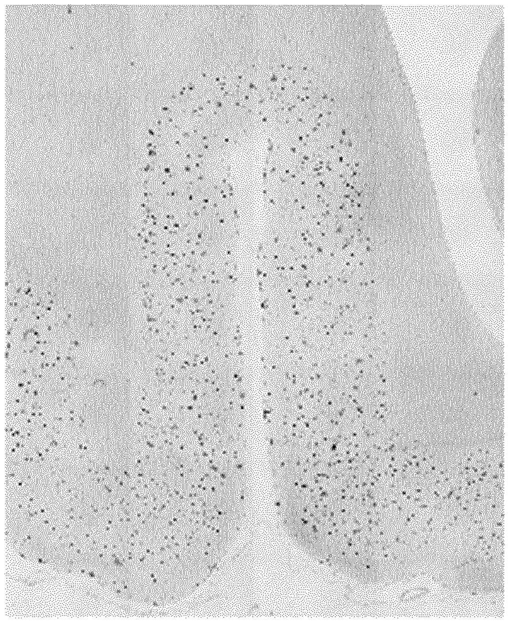

Europäisches Patentamt
European Patent Office
Office européen des brevets

# EUROPEAN SEARCH REPORT

Application Number
EP 13 18 2326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | PINGRONG YU ET AL: "18F-Labeled 2-phenylquinoxaline derivatives as potential positron emission tomography probes for in vivo imaging of [beta]-amyloid plaques", EUROPEAN JOURNAL OF MEDICINAL CHEMISTRY, vol. 57, 1 September 2012 (2012-09-01), pages 51-58, XP055093732, ISSN: 0223-5234, DOI: 10.1016/j.ejmech.2012.08.031 * Abstract; page 53, scheme 2; page 56-57 * ----- | 1-7 | INV. A61K51/04 |
| X,P | MASASHI YOSHIMURA ET AL: "Structure-Activity Relationships and in Vivo Evaluation of Quinoxaline Derivatives for PET Imaging of [beta]-Amyloid Plaques", ACS MEDICINAL CHEMISTRY LETTERS, vol. 4, no. 7, 11 July 2013 (2013-07-11), pages 596-600, XP055093656, ISSN: 1948-5875, DOI: 10.1021/ml4000707 * Abstract, page 597, cheme 1; pages 598-599 * ----- | 1-7 | TECHNICAL FIELDS SEARCHED (IPC): A61K |

The present search report has been drawn up for all claims

1

| Place of search | Date of completion of the search | Examiner |
|---|---|---|
| The Hague | 16 December 2013 | Bettio, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012226401 A
- JP 2013052369 A


### Non-patent literature cited in the description


- **G. MCKHANN et al.** Clinical diagnosis of Alzheimer's disease: Report of the NINCDS-ADRDA Work Group under the auspices of Department of Health and Human Services Task Force on Alzheimer's Disease. *Neurology,* 1984, vol. 34, 939-944
- **MENGCHAO CUI et al.** Novel quinoxaline derivatives for in vivo imaging of β-amyloid plaques in the brain. *Bioorganic & Medicinal Chemistry Letters,* 2011, vol. 21, 4193-4196
- **MENGCHAO CUI et al.** *Bioorganic & Medicinal Chemistry Letters,* 2011, vol. 21, 4193-4196
- **LISHENG MAO et al.** *Synthesis,* 2004, vol. 15, 2535-2539
- **ZHI-PING ZHUANG et al.** *J. Med. Chem,* 2003, vol. 46, 237-243